(19) Europäisches Patentamt
European Patent Office
Office européen des brevets

(11) EP 1 748 754 B1

(12) EUROPEAN PATENT SPECIFICATION

(45) Date of publication and mention of the grant of the patent:
**22.07.2009 Bulletin 2009/30**

(21) Application number: **05747197.1**

(22) Date of filing: **12.05.2005**

(51) Int Cl.:
***A61Q 3/02*** *(2006.01)* ***A61Q 5/00*** *(2006.01)*
***A61Q 5/06*** *(2006.01)* ***A61K 8/90*** *(2006.01)*

(86) International application number:
**PCT/IB2005/001307**

(87) International publication number:
**WO 2005/110351 (24.11.2005 Gazette 2005/47)**

# (54) COSMETIC COMPOSITION COMPRISING AT LEAST ONE SPECIFIC BLOCK COPOLYMER

KOSMETIKZUSAMMENSETZUNG, DIE MINDESTENS EIN BESTIMMTES BLOCKCOPOLYMER ENTHÄLT

COMPOSITION COSMETIQUE COMPRENANT AU MOINS UN COPOLYMERE SEQUENCE SPECIFIQUE

(84) Designated Contracting States:
**AT BE BG CH CY CZ DE DK EE ES FI FR GB GR HU IE IS IT LI LT LU MC NL PL PT RO SE SI SK TR**

(30) Priority: **12.05.2004 FR 0450919**

(43) Date of publication of application:
**07.02.2007 Bulletin 2007/06**

(73) Proprietor: **L'ORÉAL**
**75008 Paris (FR)**

(72) Inventors:
- **MOUGIN, Nathalie**
**F-75011 Paris (FR)**
- **JEGOU, Gwenaëlle**
**F-93190 Livry (FR)**

(74) Representative: **Le Coupanec, Pascale A.M.P.**
**Nony & Associés**
**3, rue de Penthièvre**
**75008 Paris (FR)**

(56) References cited:
**WO-A-99/47570**

- **CHO B-K ET AL: "SYNTHESIS OF LIQUID CRYSTALLINE ROD-COIL DIMERS BASED ON POLY(PROPYLENE OXIDE) COIL" POLYMER BULLETIN, SPRINGER VERLAG. HEIDELBERG, DE, vol. 44, no. 4, May 2000 (2000-05), pages 393-400, XP000945662 ISSN: 0170-0839**
- **LEE M ET AL: "LIQUID-CRYSTALLINE ROD-COIL POLYMERS BASED ON POLY(ETHYLENE OXIDE) AND THE INFLUENCE OF THE COMPLEXATION OF LICF3SO3 ON THE LIQUID-CRYSTALLINE ASSEMBLY" JOURNAL OF MATERIALS CHEMISTRY, THE ROYAL SOCIETY OF CHEMISTRY, CAMBRIDGE, GB, vol. 6, no. 7, July 1996 (1996-07), pages 1079-1086, XP000640400 ISSN: 0959-9428**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention is targeted at providing cosmetic compositions comprising at least one aqueous phase, at least one preservative and at least one specific block copolymer.

Generally, polymers are compounds frequently employed in cosmetic compositions for conferring better properties on the latter, i.e. in terms of formulation, of hold over time and/or of comfort for the users.

Thus, numerous compositions and in particular hair compositions referred to as hair styling compositions, which are provided in the form of sprays, gels or foams, comprise resins or polymers.

They are in particular acrylic polymers having high glass transition temperatures (Tg). Such polymers confer in particular increased form retention on hairstyles. However, their excessively high friability does not make it possible to guarantee prolonged hold over time of this form retention. One alternative then consists in combining them with "plasticizing" compounds which make it possible to lower the glass transition temperature. Unfortunately, the polymers then have a tendency to display a tacky effect and/or a decrease in the styling, that is to say to partially lose their mechanical properties.

Polymers also constitute important components in the field of nail varnishes. They contribute in particular to the formation of the varnish film applied at the surface of the nails. However, as for the preceding application, this protective film, which comprises the polymer, does not always prove to be sufficiently resistant to the mechanical abrasion to which varnish-coated nails are subjected in everyday life.

Consequently, there is currently no satisfactory solution available for conferring on a cosmetic composition, via polymers, properties which are simultaneously satisfactory in terms of mechanical strength and of nontacky effect.

Unexpectedly, the inventors have found that cosmetic compositions comprising block copolymers of rod-coil type make it possible specifically to meet all these requirements.

The polymers considered in the present invention are easily applied to the hair and, surprisingly, exhibit a satisfactory styling ability and are easily removed with a simple shampoo. Brushing the treated hair does not result in a powdering effect but leaves the hair soft, glossy and nontacky.

Rod-coil block copolymers are copolymers combining, on the one hand, one or more polymer blocks forming a rod, "rod block", characterized by a tendency to self-assemble into an anisotropic structure, and, on the other hand, one or more polymer blocks in the form of a random ball of wool, "coil block", subject to multiple conformations, in contrast to a rod block. These two types of blocks, rod and coil, are covalently bonded to one another.

These rod-coil block copolymers are characterized by a very high immiscibility, due to the great incompatibility of the respective rod and coil blocks, and are therefore clearly advantageous in producing microstructures and nanostructures with significant separate phases.

International application WO 99/47570 thus provides for the use of rod-coil block copolymers in a solvent which is selective for just one of the two blocks, so as to dissolve the block concerned and thus to make it possible for the copolymers of rod-coil type to self-assemble into organized mesostructures with a region of undissolved blocks and a region of dissolved blocks.

The rod blocks more particularly provided for in application WO 99/47570 are polymers incorporating several aromatic ring systems, such as quinoline, pyridine, thiophene and benzimidazole, for example, in their chemical structure. It is the $\pi$ interactions, also referred to as $\pi$-$\pi$ stacking, generated between these ring systems which make it possible to ensure the stiffness of the rod blocks. More specifically, the overlapping of the $\pi$ bonds between two aromatic groups via the delocalization of the electrons produces an energy minimum which stabilizes the structure and which results in a parallel orientation of the rod blocks. Mesostructures of this type are described in particular as advantageous, on the one hand, for encapsulating active materials of highly varied natures inside the mesostructure and, on the other hand, for forming absorption layers at the surface of a substrate, in particular in an optical device.

The present invention is based on the demonstration by the inventors that such rod-coil block copolymers can advantageously be turned to advantage in the cosmetics field in producing cosmetic compositions possessing improved properties in terms of hold over time and/or of mechanical strength.

Thus, these copolymers can be used for their mechanical properties as film-forming polymers. They can also be used, alone or as a mixture, either with other polymers, as matrix reinforcements, or with advantageous molecules, such as, for example, coloring agents and/or UV stabilizers, as vehicle for their encapsulation. These copolymers can furthermore be used as surface-active agents and/or rheological agents.

More specifically, the present invention relates, according to a first of its aspects, to a cosmetic composition comprising, in a physiologically acceptable medium:

- at least one aqueous phase,
- at least one preservative, and
- at least one block copolymer of rod-coil type comprising at least one coil polymeric block structure with a variable conformation covalently bonded to at least one rod polymeric block structure with a restricted conformation, said

block copolymer of rod-coil type being employed at concentrations capable of conferring, on the physiologically acceptable medium comprising it, a viscosity suited to the desired formulation.

According to an alternative form of the invention, the rod polymeric block incorporates, in its chemical structure, at least one, in particular at least two, aromatic ring systems, indeed even more.

According to a specific embodiment of the invention, the rod polymeric block structure with a restricted conformation is generated, in all or part and in particular completely, by π interactions, also referred to as π-π stacking, between the aromatic ring systems present in this polymeric block.

The present invention also relates, according to another of its aspects, to the use of rod-coil block copolymers in accordance with the invention as rheological agent in a cosmetic composition comprising at least one preservative and at least one aqueous phase.

The present invention also relates, according to another of its aspects, to a method for making up and/or caring for the skin, superficial body growths and/or hair comprising at least one stage of application of a cosmetic composition as defined above.

## ROD-COIL BLOCK COPOLYMER

The compositions according to the invention comprise at least one rod-coil block copolymer, also referred to as rod-b-coil block copolymer.

As specified above, these rod-coil copolymers prove to be particularly effective in creating nanoscopic arrangements in a solvent or nonsolvent medium. This is because the rod-coil molecular architecture, as well as the respective chemical natures of the two rod and coil blocks and in particular their incompatibility, involves a phase separation of the rod blocks and of the coil blocks into structures at the submicronic scale. They thus prove to be capable of self-assembling in bulk or in a solvent medium into a variety of supramolecular structures.

The formation of a specific morphology can be controlled, obviously by the nature of the blocks but also by the molecular weight, the relative lengths of the blocks, the nature of the solvent, the concentration of polymer(s), the additives, the temperature and/or the pH.

The rod-coil block copolymers according to the invention comprise at least one self-organized rod block and at least one coil block.

Within the meaning of the present invention, the term "block" is understood to mean a repeating sequence of monomer units, this repetition being at least equal to 2 units, in particular to 3 units, especially at least equal to 5 units, indeed even at least equal to 7 units.

The rod-coil block copolymers of the invention can be provided under different architectures, namely diblocks of AB type with A describing the rod block and B the coil block, triblocks of ABA or BAB, ABC type or multiblocks $(AB)_n$ or $(ABC)_n$ with C denoting a rod or coil block which is different in nature from A and B. The copolymers can also be branched polymers with a backbone composed of the rod block, or branched polymers with rod grafts, or star polymers.

The number overall mass of the rod-coil copolymers according to the invention varies generally from 700 g/mol to 1 000 000 g/mol, in particular from 1000 g/mol to 800 000 g/mol and more particularly from 2000 g/mol to 500 000 g/mol.

The number overall mass of the polymers is determined by gel permeation chromatography GPC with an eluent which is a solvent of the block copolymer. Standardization is carried out with calibration by a homopolymer standard.

The copolymers according to the invention can comprise at least one crosslinkable block. According to an alternative form of the invention, they are advantageously employed in a noncrosslinked form.

Rod-coil block copolymers prove to be particularly advantageous because of their advantageous mechanical properties at low mass and because of their low viscosity in comparison with "conventional" polymers, which have to have high molecular weights in order to obtain the necessary mechanical properties.

Furthermore, these polymers may advantageously be soluble or dispersible in cosmetic media and in particular in an aqueous, aqueous/organic or organic medium.

Within the meaning of the invention, a polymer is said to be soluble if it forms a liquid solution, at 5% by weight, in the solvent under consideration at 25°C; it is described as dispersible if, at 5% by weight in the solvent under consideration at 25°C, it forms a stable suspension of fine, generally spherical, particles with a mean size which can be less than 1 micron, can vary from 5 to 400 nm, and in particular can vary from 10 to 250 nm.

These polymers prove to be particularly advantageous in makeup compositions comprising a fatty phase and in particular fatty esters or mineral or silicone oils.

These copolymers also prove to be compatible with an application in the spray form. They are therefore generally advantageous on this account for use in the hair field.

Finally, when they have an amphiphilic nature, they can be easily carried in a water-soluble or fat-soluble medium by virtue of the block which gives them the desired solubility.

**ROD BLOCK**

The rod blocks according to the invention exhibit a persistence in trajectory, that is to say an orientational anisotropy, which implies a restricted degree of freedom of the chains.

The pseudorigid, indeed even rigid, conformation of the rod blocks is provided, in all or part and generally completely, by noncovalent interactions which impose a direction on the chain and which relate, according to the invention, to the existence of interactions of π-π or stacking type, that is to say obtained by the stacking of the units concerned. Other interactions additional to the π-π interactions may exist, such as, for example, hydrogen bonds and/or steric repulsion.

The interactions of π-π type, when sufficient in number and/or strategically placed, make it possible to significantly restrict the number of possibilities of arrangement in space with respect to one another of the various monomers constituting the rod block. Thus, the mean length of the chain, taking into account the various conformations possible, is greater.. All the restrictions imposed on the free articulation of the monomers with respect to one another have the effect, on average, of stretching the chain and thus of increasing the mean distance between the chain ends symbolized by $<R_0^2>_{rod}$, which can then satisfy the following convention:

$$<R_0^2>_{rod} = CNL^2,$$

where

- L represents the length of a monomer,
- C represents the restrictions imposed on the chain, with C greater than 1, in particular varying from 4 to 10, and
- N represents the number of monomers constituting the block.

According to a specific alternative form of the invention, the restricted conformation of the rod polymeric structure is provided, in all or part and in particular completely, by π-π interactions.

Such interactions are observed with polymers comprising one or more condensed or noncondensed aromatic rings composed of 5 to 6 carbon atoms, in particular of phenyl type, which can comprise one or more heteroatoms chosen from N, S and/or O and can carry one or more functional groups of the following types: esters, amides and/or saturated or unsaturated and linear or branched $C_1$ to $C_{30}$ alkyl groups.

These rings can, for example, be separated by double bonds, triple bonds, ester bonds, ether or thioether bonds, amide bonds, azo bonds and/or imine bonds.

Such rod blocks are described in particular by U. Scherf in Topics in Current Chemistry, vol. 204, 1999, 163-222.

More particularly, the polymeric aromatic structures constituting the rod block can be symbolized by the following general formula:

$$-[Ar]-_n$$

with:

- it being possible for n to vary from 2 to 1000, in particular from 5 to 700 and especially from 20 to 700, and
- it being possible for the Ar group(s) to represent one or more aromatic unit(s) or derivative(s) of aromatic units and it being possible for the combined aromatic units constituting said formula to be identical or different in chemical nature.

In the present application, "aromatic unit" is to be understood in the sense of the definition given by the IUPAC.

The term "derivatives of aromatic units" is understood to mean in particular, within the meaning of the invention, the compounds in which the arylene groups are substituted and in particular in which the hydrogen atoms are substituted, in all or part, by fluorine atoms, and also the ionic forms of these compounds, if appropriate in a neutralized form.

Mention may in particular be made, by way of illustration and without limitation of these Ar units, of the following groups:

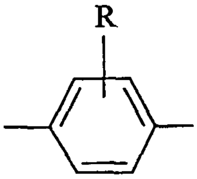 ; 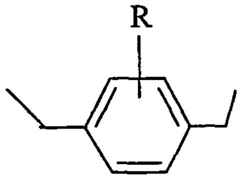 ; 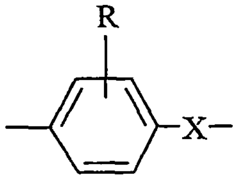 ;

;

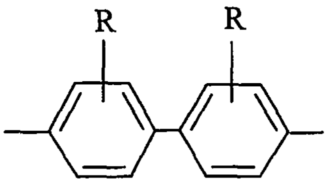 ; 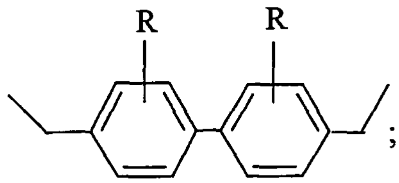 ;

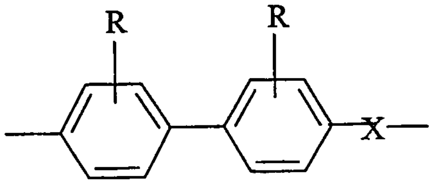

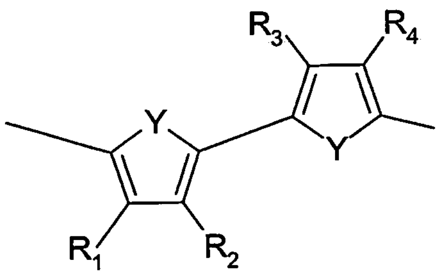

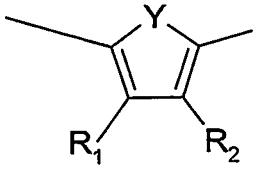 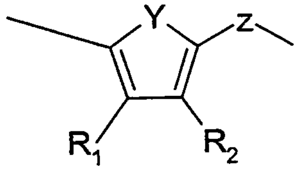

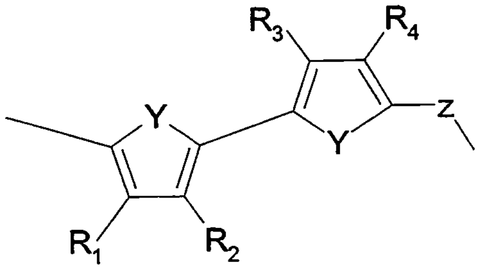

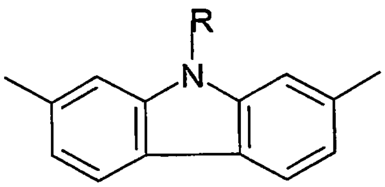 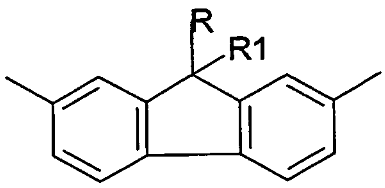

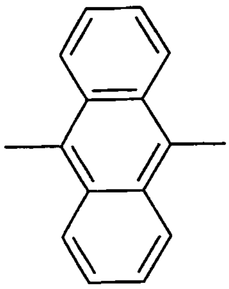 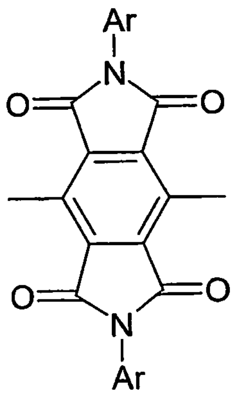

in which:

- X and Z represent, independently of one another, a unit chosen from: -O-, -NR-, -S-, -NRCO-, -OCONH-, -NHCONR-, -OCO-, -COO-, -OCOO-, -CONHNH-, -CONHNHCO-, $-SO_2-$, $-C(CH_3)_2-$, $-CH_2-$, -CR=CR-, -CR=N-, -N=N-, -N=CRAr- and - C≡C-,
- Y represents -NR-, -O- or -S-.
- R, $R_1$, $R_2$, $R_3$ and $R_4$ represent, independently of one another, a hydrogen atom, -R', -OR', -OCOR', -COOR', with R' representing a linear or branched $C_1$ to $C_{50}$, in particular $C_1$ to $C_{20}$, alkyl radical, -CN, $-N(CH_3)_2$,$-N(C_2H_5)_2$, a polycondensed or nonpolycondensed, aromatic or nonaromatic, $C_6$ to $C_{30}$ ring which can comprise one or more heteroatoms, such as, for example, O, N, S, Si, or a solubilizing group
- Ar represents an aromatic unit, chosen in particular from

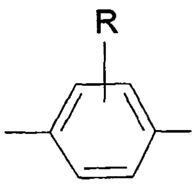 ; 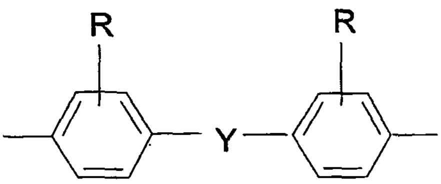 ; 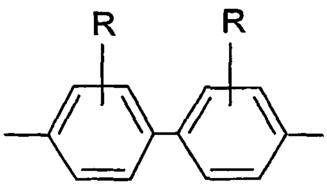

with R and Y as defined above, and their derivatives.

According to a specific embodiment, X and Z represent, independently of one another, a unit chosen from: -O-, -NH-, -S-, -NHCO-, -OCONH-, -NHCONH-, -OCO-, -COO-, - OCOO-, -CONHNH-, CONHNHCO-, $-SO_2-$, $-C(CH_3)_2-$, $-CH_2-$, -CH=CH-, $-C(CN)=CN(CH_3)_2-$, -CH=N-, -N=N- and -C≡C-, and Y represents -NH-, -O- or -S-.
More specifically, the rod block according to the invention can be composed, in all or part, of at least one unit chosen from:

- poly(para-phenylene sulfide) of formula:

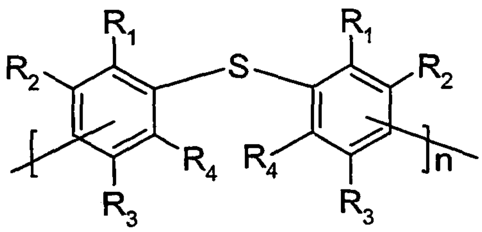

- poly(para-phenylene) of formula:

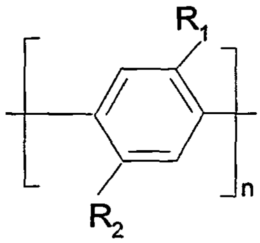

- poly(para-phenylene vinylene) of formula:

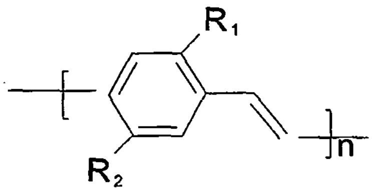

- substituted poly(para-phenylene vinylene) of formula:

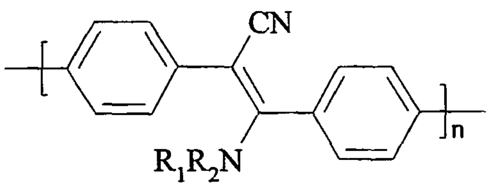

- poly(para-phenylene ethynylene) of formula:

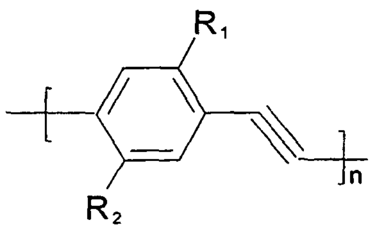

- poly(para-phenylene ether) of formula:

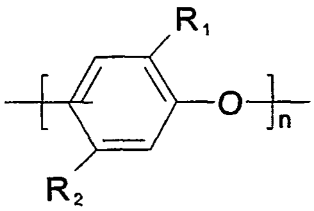

- poly(biphenylene ether) of formula:

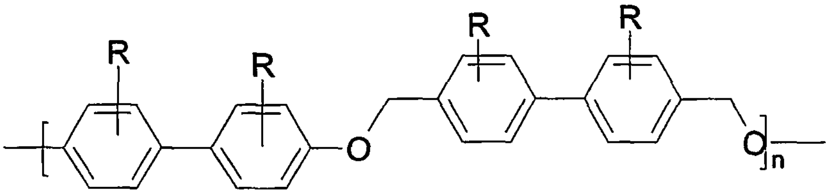

- polyanilines of formula:

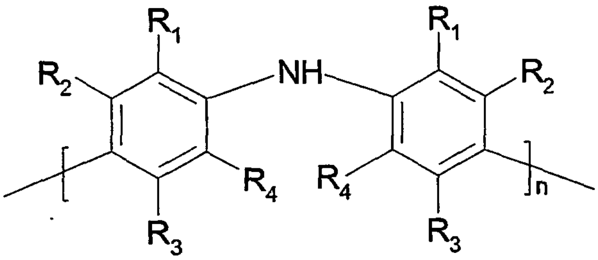

- aromatic polyazomethines of formulae:

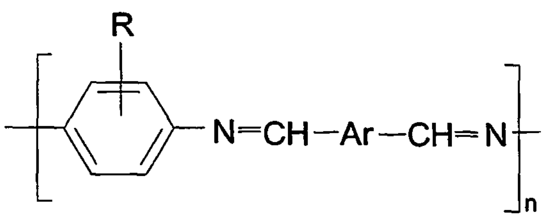

and

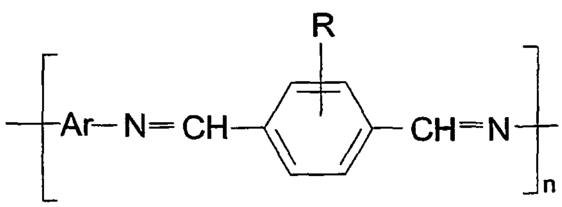

- aromatic polyamides of formulae:

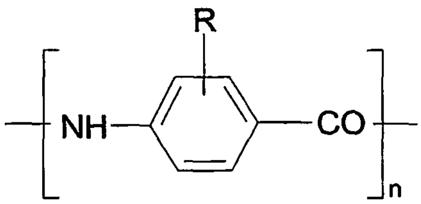

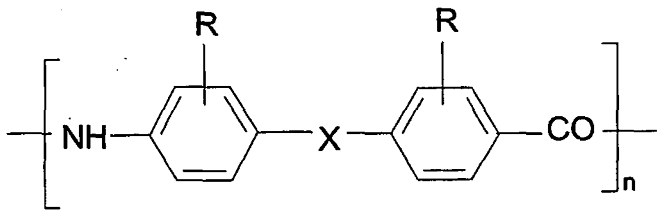

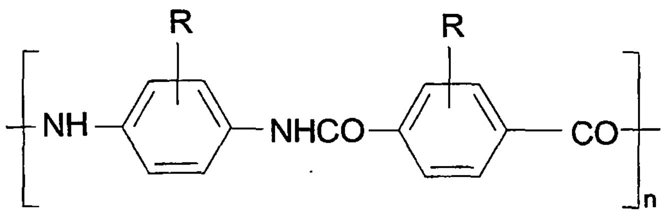

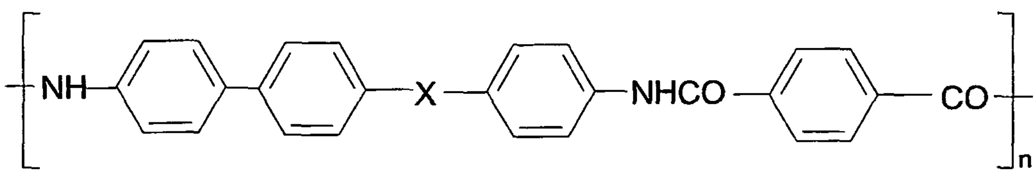

- aromatic polyhydrazides of formulae:

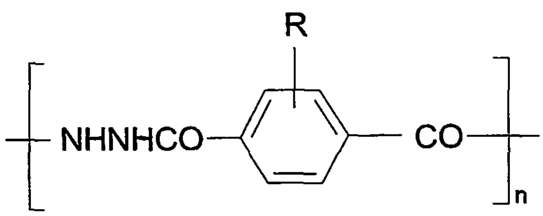

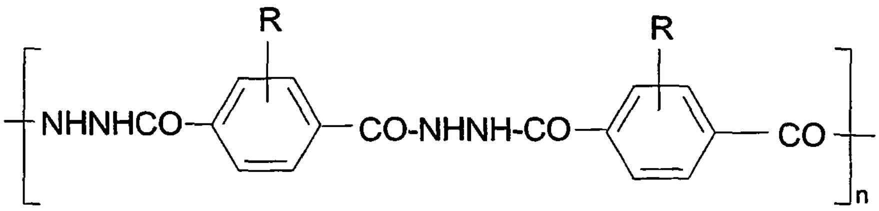

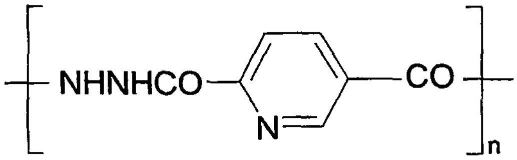

- aromatic polyesters of formulae:

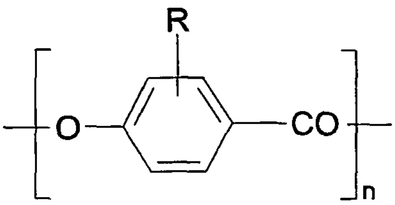

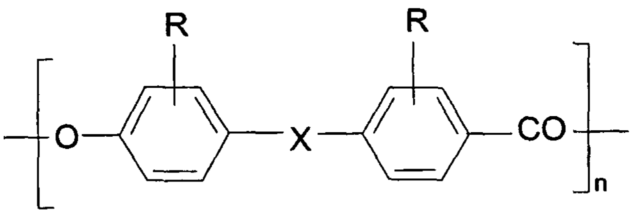

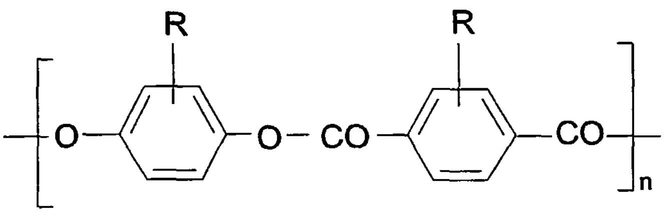

- polypyrroles of formula:

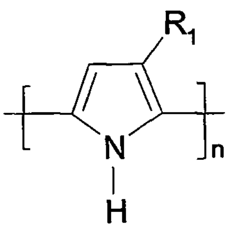

- polyfurans of formula:

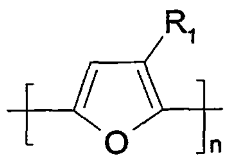

- poly(thiophene vinylene)s of formula:

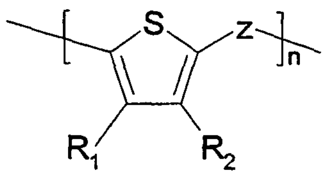

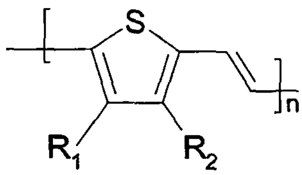

- poly(thiophene ethynylene) of formula:

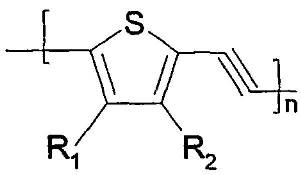

- polybithiophenes of formula:

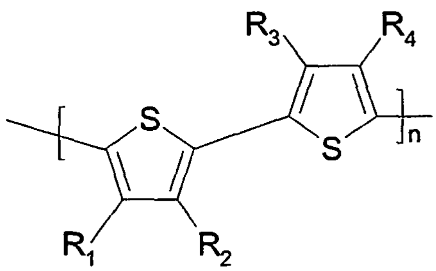

with n, Ar, R, $R_1$, $R_2$, $R_3$, $R_4$, X, Y and Z being as defined above.

As specified above, the derivatives of the abovementioned compounds can be provided in the ionic form. In this case, the R, $R_1$, $R_2$, $R_3$ and $R_4$ groups can symbolize, independently of one another, a group comprising an anionic or cationic charge which can, if appropriate, be neutralized or quaternized, in the case of the cationic charges.

The anionic groups can be neutralized with an inorganic base, such as LiOH, NaOH, KOH, $Ca(OH)_2$ or $NH_4OH$, or an organic base, such as a primary, secondary of tertiary alkylamine, such as butylamine or triethylamine. This primary,

secondary or tertiary alkylamine can comprise nitrogen and/or oxygen atoms and thus can comprise, for example, an alcohol functional group, for example such as 2-amino-2-methylpropanol or triethanolamine.

The cationic groups can be neutralized with an acid, such as sulfuric acid or hydrochloric acid, or an organic acid. This organic acid can comprise one or more carboxylic acid, sulfonic acid or phosphonic acid groups. Thus, the organic acids can be linear, branched or cyclic aliphatic acids or alternatively aromatic acids. These acids can additionally comprise one or more heteroatoms chosen from O and N, for example in the form of hydroxyl groups.

An example of an acid with an alkyl group is acetic acid $CH_3COOH$.

An example of a polyacid is terephthalic acid.

Examples of hydroxy acids are citric acid and tartaric acid.

The cationic groups can also be symbolized within the compounds in the form quaternized by compounds with a mobile halogen, such as $C_1$-$C_{12}$ alkyl chlorides or bromides and in particular methyl bromide or ethyl chloride, by sodium chloroacetate or by cyclic sulfones, such as, for example, propanesultone.

For example, the carboxylic acid or sulfonic acid functional groups can be neutralized with sodium hydroxide, 2-amino-2-methylpropanol or triethylamine. Likewise, the amine radicals can be neutralized with hydrochloric acid, acetic acid or lactic acid.

As specified above, the R, $R_1$, $R_2$, $R_3$ and $R_4$ groups can also represent, independently of one another, a solubilizing group.

The term "solubilizing group" is understood to denote any chemical unit capable of conferring on said polymer the required solubility or dispersibility in the cosmetic medium, namely, in particular, in its fatty phase or its aqueous phase.

A polymer is said to be soluble if it forms a stable solution or dispersion in the solvent or solvent mixture under consideration.

A polymer is said to be water-soluble if it forms a clear solution when it is in solution at 5% by weight in water at 25°C.

A polymer is said to be water-dispersible if, at 5% by weight in water at 25°C, it forms a stable suspension of fine, generally spherical, particles. The mean size of the particles constituting said dispersion can be less than 1 μm and more generally varies between 5 and 400 nm, in particular from 10 to 250 nm. These particle sizes are measured by light scattering.

Mention may be made, by way of representation of the solubilizing groups capable of contributing solubility or dispersibility in water, for example, of the following units:

- carboxyl (-COOH) or carboxylate ($-COO^-M^+$) radical,
- sulfo ($-SO_3H$) or sulfonate ($-SO_3^-M^+$) radical,
- phosphono ($-PO(OH)_2$) or phosphonate ($-PO(O^-)_2\ M^+{}_2$) radical,
  with M representing, for example, an alkali metal, such as sodium or potassium, or an organic amine, such as a primary, secondary or tertiary amine, - amine radical: $-NH_2$, -NHR or $-NR_1R_2$, or one of their organic or inorganic salts,
- quaternary ammonium radical, such as: $-N^+R'_3\ Z^-$ with Z representing, for example, a Br or Cl atom or a ($C_1$-$C_4$) alkyl-$OSO_3$ radical,
  and R' representing, for example, identical or different, linear or branched, $C_1$ to $C_{20}$ alkyl groups,
- hydroxyl radical,
- poly($C_2$-$C_3$ alkylene oxide) radical, and
- their mixtures.

As specified above, these radicals may or may not be employed in a neutralized form.

According to a specific alternative form, the rod block has one or more solubilizing groups capable of rendering it dispersible or soluble in an aqueous phase.

More particularly, these solubilizing groups can be chosen from the following units: -COOH, $-CH_2COOH$, $-(CH_2)_2COOH$, - $(CH_2)_3COOH$, -OH, $-CH_2OH$, - $(CH_2)_6OH$, $-(CH_2)SO_3H$, $-(CH_2)_2SO_3H$, $-(CH_2)_3SO_3H$, $-O(CH_2)_3SO_3H$, - $(CH_2)_2N(CH_3)(CH_3)$, $-N(CH_3)(CH_3)$, - $O(CH_2)_3N(CH_2CH_3)_2$, $-(CH_2)_2N(CH_2CH_3)_2$, - $(CH_2)_2O]_xCH_2CH_2OH$, - $[(CH_2)_2O]_xCH_2CH_2OCH_3$ or - $[(CH_2)_2]_xCH_2CH_2OCH_2CH_3$, with x symbolizing a mean number of between 0 and 200, and their sodium and amine salts, in particular their triethylamine or tributylamine salts.

The solubilizing groups can also be groups contributing solubility or dispersibility in a fatty phase composed in particular of fatty ester(s) or of mineral and/or silicone oil (s) .

As such, the following are suitable in particular according to the invention:

- siloxane derivatives, such as polyalkylsiloxanes, in particular polydimethylsiloxanes (PDMSs), polyalkylarylsiloxanes, polarylsiloxanes, alkylsiloxanes, alkylarylsiloxanes or arylsiloxanes, which may or may not be modified, and which may or may not comprise fluorines, alkoxy or alcohol functional groups or poly($C_2$ to $C_3$ alkylene oxide) chains,

- polyethylene, polypropylene, polybutadiene, which may or may not be hydrogenated, poly(ethylene butylene) or polyisobutylene,
- linear or branched $C_8$ to $C_{30}$ alkyls, and
- their mixtures.

These solubilizing groups can be chosen particularly from polyalkylsiloxanes, such as polydimethylsiloxanes (PDMSs), polydimethylsiloxanes comprising poly($C_2$-$C_3$ alkylene oxide) units, polydimethylsiloxanes comprising poly ($C_2$-$C_3$ alkylene oxide) units and lauryl units, polydimethylsiloxanes comprising perfluorinated units, polyalkylarylsiloxanes, such as, for example, polymethylphenylsiloxanes, nonhydrogenated polybutadienes, poly(ethylene butylene), linear or branched $C_{12}$, $C_{14}$, $C_{16}$, $C_{18}$ or $C_{22}$ alkyls, and their mixtures.
All the abovementioned solubilizing radicals can be connected to the ring via a spacer groups, such as, for example, an -R"-, -OR"-, -OCOR"- or -COOR"- radical with R" representing a linear or branched $C_1$-$C_{20}$ alkylene radical.
A person skilled in the art will choose the various groups such as to avoid any risk of chemical incompatibility between them.
More particularly, the rod blocks can be chosen from:

- polyarylenes, poly (arylene vinylene) s, poly(arylene ethynylene)s or poly(arylene ether)s and, among these, poly (para-phenylene)s, poly(para-phenylene vinylene)s, poly(para-phenylene ethynylene)s, poly(para-phenylene ether) s, poly(para-phenylene sulfide)s and poly(biphenylene ether)s,
- polyquinolines, polyquinoxalines, polypyridines, poly(pyridine vinylene) s, poly (naphthylene vinylene) s, polythiophenes, poly(thiophene vinylene)s, poly(thiophene ethynylene)s, polybisthiophenes, polyfurans, polypyrroles, polyanilines, polybenzimidazoles, polybenzothiazoles, polybenzoxazoles, polybenzobisazoles, aromatic polyamides, aromatic polyhydrazides, aromatic polyazomethines, aromatic polyesters, aromatic polyimides, polycarbazoles, polyfluorenes or polyanthracenes, and
- their copolymers.

In particular, they can be polyarylenes, poly (arylene vinylene)s, poly(arylene ethynylene)s, poly(arylene ether) s, polyanilines, polypyrroles or polythiophenes, and their derivatives and mixtures.
Rod blocks such as:

- poly[2,5-bis(4-methoxyphenyl)oxycarbonyl]styrene,
- poly(4'-oxybiphenyl),
- poly(phenylene benzobisthiazole),
- poly(1,4-(2,5-dihexylphenylene)),
- poly(1,4-(2,5-dihexylphenylene) ethynylene),
- poly(1,4-(2,5 dihexylphenylene) vinylene),
- poly(3-dodecylthiophene),
- poly(3-octylthiophene),
- poly[1,4-(2,5-di(polydimethylsiloxane)phenylene)], and
- their copolymers, are very particularly suitable for the invention.

In the context of the present invention, water-soluble or water-dispersible rod block polymers are particularly advantageous.
According to a specific alternative form of the invention, the number mass of the rod blocks varies from 200 g/mol to 1 000 000 g/mol, in particular from 250 g/mol to 800 000 g/mol and more particularly from 250 g/mol to 500 000 g/mol.
According to a specific alternative form of the invention, the rod blocks are present in a proportion of at least 10%, in particular of at least 15%, especially of at least 30%, and in particular of at most 90%, especially of at most 85%, indeed even of at most 80%, by weight, with respect to the total weight of the copolymer.
This ratio can in particular be conditioned by the solubility desired. Thus, when the coil block is soluble in the desired medium whereas the rod block is not soluble, the proportion of the first type of coil block can advantageously be adjusted to a high value.

## COIL BLOCK

In contrast to the rod blocks described above, a coil block is composed of a homopolymer or copolymer, the chain of which has a random variable geometrical conformation. The degrees of freedom of the chemical bonds between the monomers constituting the chain are markedly more numerous in the case of a coil block than in the case of a rod

block, which results in a markedly higher number of possible conformations.

In contrast to the rod block, the mean distance between the chain ends in the coil block, namely $<R_0^2>_{coil}$, satisfies the convention:

$$<R_0^2>_{coil} = NL^2$$

with N and L as defined above.

The blocks composed of one or more homopolymers or copolymers deriving from the radical polymerization of monomers comprising ethylene, vinyl, allyl, (meth)acrylate and/or (meth)acrylamide units, and their derivatives, are very particularly suitable for the invention as coil block.

For example, vinyl/(meth)acrylate, vinyl/(meth)acrylamide, vinyl/(meth)acrylate/methacrylamide, olefin/vinyl and (meth)acrylate/(meth)acrylamide copolymers are suitable for the invention.

Mention may more particularly be made, by way of illustration of these polymers, of the homopolymers or copolymers based on vinyl acetate, on styrene, on vinylpyrrolidone, on vinylcaprolactam, on poly (ethylene oxide) (meth) acrylate, on stearyl (meth)acrylate, on lauryl (meth)acrylate, on vinyllaurate, on butyl (meth)acrylate, on ethylhexyl (meth) acrylate, on crotonic acid, on (meth)acrylic acid, on maleic anhydride, on styrenesulfonic acid, on dimethyldiallylamine, on vinylpyridine, on dimethylaminoethyl (meth)acrylate or on dimethylaminopropyl-(meth)acrylamide, and their salts.

They can also be:

- polycondensates of polyurethane and/or polyurea type, aliphatic polyesters, aliphatic polyamides or their copolymers, such as, for example, polyurethane/urea and polyester/amide,
- polymers obtained by ring opening, such as polyethers of poly(ethylene oxide) or poly(propylene oxide) type, and their poly(ethylene oxide)/poly(propylene oxide) copolymers, polyesters, such as polycaprolactone, poly(lactic acid) or poly(hydroxyalkanoates), or polyoxazolines, such as poly(2-methyloxazoline) or poly(2-ethyloxazoline),
- siloxane (co)polymers, such as, for example, polyalkoxysiloxanes (in particular polydimethylsiloxanes), polyalkylarylsiloxanes or polyarylsiloxanes, which may or may not be modified and which may comprise fluorines, hydroxyls, alkoxy functional groups and/or poly($C_2$-$C_3$ alkylene oxide) chains,
- polymers obtained by metathesis, such as poly(norbornene) and its copolymers,
- polymers obtained by cationic polymerization, such as poly(vinyl alkyl ether)s, such as, for example, poly(vinyl methyl ether)s,
- copolymers of these various types of polymer, such as, for example, polysiloxane/poly(ethylene oxide)s, and
- their salts and their derivatives.

These polymers can, if appropriate, be functionalized so as to confer on them a soluble or dispersible nature, in particular in one of the media of the cosmetic composition in which they are intended to be formulated, such as, for example, in water, alcohols, carbonaceous oils, esters, fluorinated oils, silicone oils and/or any other fatty substance.

Blocks which are soluble or dispersible in water and/or ethanol and which can comprise hydrophilic nonionic, anionic and/or cationic units are more particularly suitable for the invention as coil block. Polymers comprising anionic or cationic groups can be employed in a form neutralized, or not.

The neutralization of these groups can be identical or different from that discussed previously for the rod blocks. The anionic groups can be neutralized, for example, with an inorganic base, such as LiOH, NaOH, KOH, $Ca(OH)_2$ or $NH_4OH$, or an organic base as defined above. With regard to the neutralization of the cationic groups, it can be carried out with an acid, such as sulfuric acid or hydrochloric acid, or an organic acid as defined above. Moreover, the cationic groups can be quaternized as defined above.

Mention may in particular be made, by way of representation of polymers comprising nonionic units, of polymers based on ethylene oxide, on methyloxazoline, on ethyloxazoline, on vinylpyrrolidone, on vinylcaprolactam and on poly (ethylene oxide) (meth)acrylate, poly(ethylene oxide)s or poly(ethylene oxide)/poly(propylene oxide) copolymers.

Mention may more particularly be made, as anionic polymers, of radical homopolymers or copolymers based on crotonic acid, on (meth)acrylic acid, on maleic anhydride or on styrenesulfonic acid, sulfonic aliphatic polyesters, sulfonic aliphatic polyamides and their salts.

Mention may more particularly be made, as cationic polymers, of radical homopolymers or copolymers based on dimethyldiallyamine, on vinylpyridine, on dimethylaminoethyl (meth)acrylate or on dimethylaminopropyl(meth)acrylamide, tertiary amine aliphatic polyesters, tertiary amine aliphatic polyamides and their salts.

As regards the synthesis of the coil blocks, reference may be made in particular to the document "chime et physicochimie des polymères [Chemistry and Physical Chemistry of Polymers]", published by Dunod, 2002.

Fat-soluble coil copolymers are also suitable for the invention.

Mention may be made, by way of representation of water-insoluble and fat-soluble polymers forming the coil block, of:

- polydimethylsiloxanes (PDMSs),
- polydimethylsiloxanes comprising poly($C_2$-$C_3$ alkylene oxide) units, polydimethylsiloxanes comprising poly($C_2$-$C_3$ alkylene oxide) units and lauryl units, polydimethylsiloxanes comprising perfluorinated units which are soluble in silicones or in the silicone derivatives,
- polyalkylarylsiloxanes, such as polymethylphenylsiloxanes,
- polybutadienes, which may or may not be hydrogenated, polyisobutylenes and (ethylene/butylene) copolymers which are soluble in mineral oils,
- alkyd polyesters with $C_6$ to $C_{30}$ carbon chains,
- polyamides with $C_6$ to $C_{30}$ carbon chains,
- vinyl polymers based on vinyl laurate,
- poly (meth) acrylates based on stearyl (meth) acrylate, on isobornyl (meth)acrylate, on cyclohexyl (meth)acrylate, on butyl (meth)acrylate, on ethylhexyl (meth)acrylate, or on lauryl (meth)acrylate.

In the copolymers according to the invention, the number mass of the coil block(s) can vary from 500 g/mol to 1 000 000 g/mol, in particular from 500 g/mol to 800 000 g/mol and more particularly from 500 g/mol to 500 000 g/mol.

Mention may very particularly be made, by way of illustration of the rod-coil (also known as rod-b-coil) block copolymers according to the invention, of:

- diblocks: rod-b-coil
  - polydimethylsiloxane-b-poly(p-phenylene ether),
  - polydimethylsiloxane-b-poly[2,5-bis(4-methoxyphenyl)-oxycarbonyl]styrene,
  - poly(ethylene/butylene)-b-poly(4'-oxybiphenyl),
  - (alkyd polyester with $C_{12}$ carbon chains)-b-poly(biphenyl-4'-biphenylcarboxylate),
  - poly(vinyl laurate)-b-poly(phenylene benzobisthiazole),
  - polyisoprene-b-poly(1,4-(2,5-dihexylphenylene)),
  - poly(dimethylsiloxane)-b-poly(2,5-(3-(ethoxyethyl)-thiophene)),
  - polydimethylsiloxane-b-poly(3-dodecylthiophene),
  - poly(vinyl laurate)-b-poly(3-octylthiophene),
  - poly(vinyl laurate)-b-poly[1,4-(2,5-di(polydimethylsiloxyl)phenylene)],
- triblocks: coil-b-rod-b-coil
  - polydimethylsiloxane-b-poly(p-phenylene ether)-b-polydimethylsiloxane,
  - polydimethylsiloxane-b-poly(1,4-(2,5-dihexylphenylene) ethynylene)-b-polydimethylsiloxane,
  - poly(ethylene/butylene)-b-poly(1,4-(2,5-dihexylphenylene) ethynylene)-b-poly(ethylene/butylene),
  - (alkyd polyester with $C_{12}$ carbon chains)-b-poly(biphenyl-4'-biphenylcarboxylate)-b-(alkyd polyester with $C_{12}$ carbon chains),
  - poly(vinyl laurate)-b-poly(4'-oxybiphenyl)-b-poly(vinyl laurate),
  - poly(oleyl acrylate)-b-poly(2,5-(hexylthiophene))-b-poly(oleyl acrylate),
  - polydimethylsiloxane-b-poly(3-dodecylthiophene)-b-polydimethylsiloxane,
- or rod-b-coil-b-rod triblocks resulting from the preceding structures, such as, for example: poly(1,4-(2,5-dihexylphenylene) ethynylene)-b-poly(ethylene/butylene)-b-poly(1,4-(2,5-dihexylphenylene) ethynylene).

The following are also suitable for the invention:

- diblocks: rod-b-coil
  - poly(ethylene oxide)-b-poly(p-phenylene ether),
  - poly(ethylene oxide)-b-poly[2,5-bis(4-methoxyphenyl)-oxycarbonyl]styrene,
  - poly(ethylene oxide)-b-poly(4'-oxybiphenyl),
  - polyvinylcaprolactam-b-poly(biphenyl-4'-biphenylcarboxylate),
  - polyvinylcaprolactam-b-poly(phenylene benzobisthiazole),
  - poly(acrylic acid)-b-poly(1,4-(2,5-dihexylphenylene)),

- polystyrenesulfonate-b-poly(1,4-(2,5-dihexylphenylene)),
- poly(acrylic acid-co-methyl methacrylate)-b-poly(1,4-(2,5-dihexylphenylene)),
- poly(acrylic acid-co-butyl acrylate)-b-poly(phenylene benzobisthiazole),
- polydimethylsiloxane-b-poly(1,4-(2,5-dicarboxybiphenyl)),
- poly(ethylene glycol)-b-poly(1,4-(2,5-dicarboxybiphenyl)),
- polydimethylsiloxane-b-poly(2,5-(3-carboxythiophene)),
- poly(ethylene glycol)-b-poly(2,5-(3-carboxythiophene)),
- poly(ethylene glycol)-b-poly(2,5-(3-ethoxyethyl)-thiophene)),
- and their sodium or amine salts,

• triblocks: coil-b-rod-b-coil

- poly(acrylic acid)-b-poly(1,4-(2,5-dihexylphenylene))-b-poly(acrylic acid),
- polystyrenesulfonate-b-poly(1,4-(2,5-dihexylphenylene))-b-polystyrenesulfonate,
- poly(acrylic acid-co-MMA)-b-poly(1,4-(2,5-dihexylphenylene))-b-poly(acrylic acid-co-MMA),
- poly(acrylic acid-co-butyl acrylate)-b-poly(phenylene benzobisthiazole)-b-poly(acrylic acid-co-butyl acrylate),
- poly(ethylene glycol)-b-poly(1,4-(2,5-dicarboxybiphenyl))-b-poly(ethylene glycol),
- poly(ethylene oxide)-b-poly(p-phenylene ether)-b-poly(ethylene oxide),
- poly(ethylene oxide)-b-poly(1,4-(2,5-dihexylphenylene) ethynylene)-b-poly(ethylene oxide),
- poly(ethylene glycol)-b-poly(2,5-(3-carboxythiophene))-b-poly(ethylene glycol),
- poly(acrylic acid-co-butyl acrylate)-b-poly(2,5-(3-(ethoxyethyl)thiophene))-b-poly(acrylic acid-co-butyl acrylate),
- polydimethylsiloxane-b-poly(2,5-(3-(ethoxyethyl)-thiophene))-b-polydimethylsiloxane,
- polydimethylsiloxane-b-poly(1,4-(2,5-dicarboxybiphenyl))-b-polydimethylsiloxane,
- poly(ethylene/butylene)-b-poly(1,4-(2,5-dicarboxybiphenyl))-b-poly(ethylene/butylene), and
- their sodium or amine salts,

• rod-b-coil-b-rod triblocks resulting from the preceding structures, such as, for example:

- poly(p-phenylene ether)-b-(ethylene oxide)-b-poly(p-phenylene ether),
- poly(2,5-(3-carboxythiophene))-b-polydimethylsiloxane-b-poly(2,5-(3-carboxythiophene)), and
- their sodium or amine salts.

All the combinations of these various blocks may also be suitable.
The following rod-coil block copolymers:

• diblocks: rod-b-coil

- polydimethylsiloxane-b-poly[2,5-bis(4-methoxyphenyl)-oxycarbonyl]styrene,
- poly(ethylene/butylene)-b-poly(4'-oxybiphenyl),
- alkyd polyester with $C_{12}$ carbon chains-b-poly(biphenyl-4'-biphenylcarboxylate),
- poly(vinyl laurate)-b-poly(phenylene benzobisthiazole),
- polyisoprene-b-poly(1,4-(2,5-dihexylphenylene)),

• triblocks: coil-b-rod-b-coil

- polydimethylsiloxane-b-poly(1,4-(2,5-dihexylphenylene) ethynylene)-b-polydimethylsiloxane,
- poly(ethylene/butylene)-b-poly(1,4-(2,5-dihexylphenylene) ethynylene)-b-poly(ethylene/butylene),
- alkyd polyester with $C_{12}$ carbon chains-b-poly(biphenyl-4'-biphenylcarboxylate)-b-alkyd polyester with $C_{12}$ carbon chains,
- poly(vinyl laurate)-b-poly(4'-oxybiphenyl)-b-poly(vinyl laurate),
- poly(oleyl acrylate)-b-poly(2,5-(hexylthiophene))-b-poly(oleyl acrylate), and
- their salts or derivatives,

are very particularly suitable for the invention.
More particularly, the rod-coil block polymers are employed in a noncrosslinked form.
The rod-coil block polymers according to the invention can be obtained by various synthetic routes.
The synthetic route can be a reaction between the two preformed rod and coil precursors which involves the reaction by condensation between a functional end of a rod block and a functional end of coil block. The synthetic route

can in particular relate to the formation of C-C, C=C or C≡C bonds.

According to another alternative form, the formation of the two blocks constituting the rod-coil block copolymer can be carried out in situ in the presence of the other block already constructed. For example, if the coil block is a polymer which can be obtained by the radical route, it is sufficient to functionalize one of the ends of the rod block with a functional group capable of initiating the controlled radical polymerization of the monomers under consideration for the coil block. Likewise, the formation of the rod block can be carried out in situ in the presence of a coil block and on a reactive functional group of this block.

Depending on the functionality of the precursors and the distribution of the functional groups, diblocks, triblocks, multiblocks, branched polymers or star polymers may be obtained.

For example, in the specific case where the condensation involves a polymerization or several successive carbon-carbon coupling reactions by reaction of monomers of type A-X-A with B-X'-B monomers or of several monomers of A-X-B type, with both X and X' being defined as X above, use may be made of one of the following coupling techniques:

- Stille coupling, which is a coupling involving Pd-based catalysis between an aromatic substituted by an $-SnR_3$ functional group and a halogenated aromatic derivative,
- Suzuki coupling, which is a coupling between a halogen functional group (for example bromine) and a boronic acid catalyzed by a palladium complex,
- Sonagashira-Heck coupling, which derives from the formation of a bond between an acetylene functional group on an aromatic and an aromatic functional group substituted by a halogen atom (bromine or iodine) catalyzed by complexes of Cu and Pd and of a base. This coupling is in particular illustrated below for the synthesis of a rod-coil diblock polymer composed of a poly(phenylene ethynylene) rod by the protocol below,

    a) functionalization of the rod by an ester functional group, which is carried out by synthesis in the presence of:

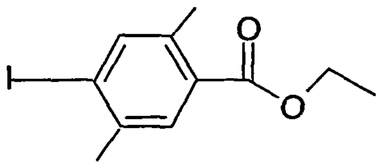

    and

    b) preparation of the rod-coil by conversion of the ester end to an acid, followed by esterification between the acid-terminated rod and the OH-terminated coil, for example:

$$HO-(CH_2CH_2O)_nH$$

    according to the reference Macromol. Rapid Commun., 1998, 19, 275-281.

This condensation can also be carried out by the formation of a C=C double bond according to the Wittig-Horner reaction, namely a coupling of aromatic aldehyde and phosphonate of aromatic esters, for example prepared from a $CH_2Br$-functionalized aromatic derivative with $P(OR)_3$, in particular $P(OEt)_3$.

This technique is illustrated below, with reference to Advanced Materials, 2001, 13, No. 18, page 1363, for the synthesis of a rod-coil triblock copolymer, the rod structure of which is composed of poly(phenylene vinylene) (PPV) and the coil structure of which is a poly(ethylene oxide) like:

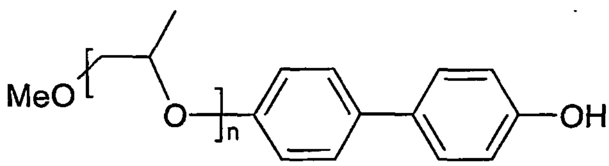

This condensation can also be carried out by:

- formation of C=N (imine) bonds by a condensation reaction between a primary or secondary amine and an aldehyde,
- formation of ether, ester, amide, ketone or amine bonds according to the standard methods described, for example, in Advanced Organic Chemistry, M.B. Smith and J. March, Wiley Interscience, 5th edition, and according to the protocol described in Macromolecules, 1999, 32, 7688-7691 and illustrated as follows:

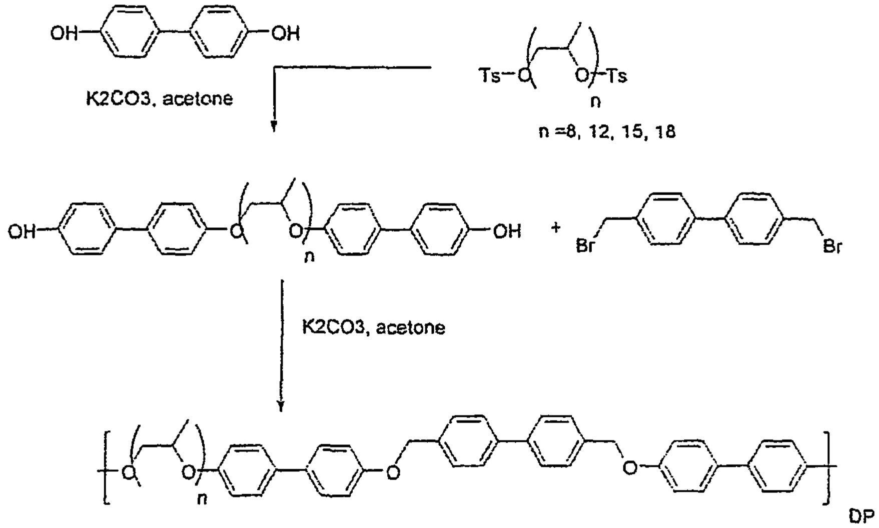


- Chemical or electrochemical oxidation, such as, for example, the polycondensation of dihalogenated thiophene by catalysis with nickel or palladium complexes. This technique may require the prior functionalization of the rod and/or coil units by addition of a monomer comprising the desired functional group.

Another type of specific coupling can be carried out by aromatic ring formation. This type of coupling is required in particular for polyphenylquinolines. In this specific case, the rod-block coupling is carried out by formation of a quinoline functional group between an acetyl-terminated coil and a polyquinoline rod block, according in particular to a protocol as follows:

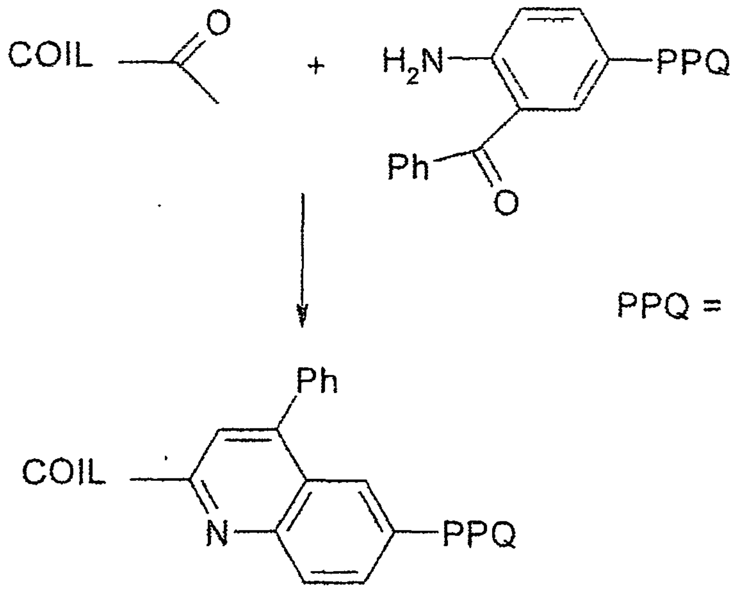

PPQ = polyphenylquinoline

In the cosmetic compositions according to the invention, the rod-coil block copolymers are employed at concentrations compatible with self-assembling in the form of a nanostructure or alternatively as rheological agent, that is

to say an agent capable of conferring, on the physiologically acceptable medium comprising it, a viscosity suited to the desired formulation.

These concentrations are generally concentrations greater than the critical micelle concentration or than the critical vesicle concentration. These critical micelle or critical vesicle concentrations characterize the concentrations below which the copolymer exists as an individual molecule or as a chain in the solution and above which it is present therein as an aggregated entity. These critical concentration values vary between $10^{-9}$ mol/l and $10^{-4}$ mol/l.

The concentrations of copolymers varying from 0.5 to 90% by weight, in particular from 0.7 to 85% by weight and more particularly from 0.8 to 75% by weight, of copolymer(s) with respect to the total weight of the composition, are very particularly suitable.

## PHYSIOLOGICALLY ACCEPTABLE MEDIUM

The term "physiologically acceptable medium" is understood to denote a nontoxic medium capable of being applied to the skin, lips or superficial body growths of human beings. The physiologically acceptable medium is generally suited to the nature of the substrate on which the composition has to be applied and to the condition under which the composition is intended to be packaged, in the case in point fluid or nonfluid at ambient temperature and at atmosphere pressure.

## AQUEOUS PHASE

The composition according to the invention comprises at least one aqueous medium, constituting an aqueous phase, which can in particular form the continuous phase of the composition.

The aqueous phase can be composed essentially of water or can be composed of a mixture of water and of at least one water-miscible organic solvent (miscibility in water of greater than 50% by weight at 25°C).

Thus, the composition according to the invention can comprise at least one organic solvent medium comprising at least one organic solvent which is volatile at ambient temperature. These organic solvents are more particularly considered when the cosmetic composition is intended for application to the nails.

Mention may be made, as organic solvent which is volatile or nonvolatile at ambient temperature, of:

- ketones which are liquid at ambient temperature, such as methyl ethyl ketone, methyl isobutyl ketone, diisobutyl ketone, isophorone, cyclohexanone or acetone;
- alcohols which are liquid at ambient temperature, such as ethanol, isopropanol, butanol, diacetone alcohol, 2-butoxyethanol or cyclohexanol;
- glycols which are liquid at ambient temperature, such as ethylene glycol, propylene glycol, pentylene glycol or glycerol;
- propylene glycol ethers which are liquid at ambient temperature, such as propylene glycol monomethyl ether, propylene glycol monomethyl ether acetate or dipropylene glycol mono(n-butyl) ether;
- short-chain esters (having from 3 to 8 carbon atoms in total), such as ethyl acetate, methyl acetate, propyl acetate, butyl acetate, an aryl acetate or isopentyl acetate;
- alkanes which are liquid at ambient temperature, such as decane, heptane, octane, dodecane, cyclohexane or isododecane; and
- their mixtures.

The abovementioned organic compounds can be present in a proportion of 0.5 to 99% by weight with respect to the total weight of the composition.

When the physiologically medium comprises a significant amount of organic phase, the latter can be present in a proportion of 30 to 99% by weight and in particular of 60 to 90% by weight, with respect to the total weight of the composition.

The aqueous phase (water and optionally the water-miscible organic solvent) can be present at a content ranging from 1 to 95% by weight, in particular ranging from 3 to 80% by weight and especially ranging from 5 to 60% by weight, with respect to the total weight of the composition.

## PRESERVATIVES

As specified above, the compositions according to the invention comprise at least one preservative.

Use may be made, as preservatives, of any preservative commonly used in the field under consideration, such as, for example, esters of para-hydroxybenzoic acid, also known as Parabens®, phenoxyethanol, compounds which release formaldehyde, such as, for example, imidazolidinylurea or diazolidinylurea, sodium benzoate, chlorhexidine

digluconate, alkytrimethylammonium bromide, such as myristyltrimethylammonium bromide (CTFA name: myrtrimonium bromide), dodecyltrimethylammonium bromide, hexadecyltrimethylammonium bromide and their mixtures, such as the mixture sold under the name Cetrimide® by FEF Chemicals, or polymer quaternary ammonium salts, such as polyquaternium-10.

The preservative is present at a content sufficient to produce the expected effect, also described as "quantity sufficient for" (q.s. for). This amount is, of course, capable of varying according to the nature of the preservative. Generally, the preservative can be present in a proportion of 0.001 to 10% by weight, in particular in a proportion of 0.1 to 5% by weight and especially in a proportion of 0.2 to 3% by weight, with respect to the total weight of the composition.

## FATTY PHASE

The composition according to the invention can additionally comprise a fatty phase which can comprise in particular at least one fatty substance which is liquid at ambient temperature (25°C) and at atmospheric pressure and/or at least one fatty substance which is solid at ambient temperature and at atmospheric pressure, such as waxes, pasty fatty substances, gums and their mixtures. The fatty phase can additionally comprise gelling and structuring agents for oils of organic nature and/or lipophilic organic solvents.

The fatty phase of the composition according to the invention can comprise in particular, as liquid fatty substance, at least one volatile or nonvolatile oil or one of their mixtures.

The term "volatile oil" is understood to mean, within the meaning of the invention, any oil capable of evaporating on contact with the skin in less than one hour, at ambient temperature and atmospheric pressure. The volatile oils of the invention are volatile cosmetic oils which are liquid at ambient temperature and which have a non-zero vapor pressure, at ambient temperature and atmospheric pressure, ranging in particular from 0.01 to 300 mmHg (1.33 Pa to 40 000 Pa) and preferably of greater than 0.3 mmHg (30 Pa).

The term "nonvolatile oil" is understood to mean an oil which remains on the skin at ambient temperature and atmospheric pressure for at least several hours and which has in particular a vapor pressure of less than 0.01 mmHg (1.33 Pa).

These volatile or nonvolatile oils can be hydrocarbon oils, in particular of vegetable origin, silicone oils, or their mixtures. The term "hydrocarbon oil" is understood to mean an oil mainly comprising hydrogen and carbon atoms and optionally oxygen, nitrogen, sulphur and/or phosphorus atoms.

The volatile hydrocarbon oils can be chosen from hydrocarbon oils having from 8 to 16 carbon atoms and in particular branched $C_8$-$C_{16}$ alkanes, such as $C_8$-$C_{16}$ isoalkanes of petroleum origin (also known as isoparaffins), for example isododecane (also known as 2,2,4,4,6-pentamethylheptane), isodecane, isohexadecane and, for example, the oils sold under the trade names of Isopars® or Permetyls®, branched $C_8$-$C_{16}$ esters, such as isohexyl neopentanoate, and their mixtures. Other volatile hydrocarbon oils, such as oil distillates, in particular those sold under the name Shell Solt® by Shell, can also be used.

Use may also be made, as volatile oils, of volatile silicones, such as, for example, volatile linear or cyclic silicone oils, in particular those having a viscosity ≤ 8 centistokes ($8 \times 10^{-6}$ $m^2/s$), having in particular from 2 to 7 silicon atoms, these silicones optionally comprising alkyl or alkoxy groups having from 1 to 10 carbon atoms. Mention may in particular be made, as volatile silicone oil which can be used in the invention, of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, dodecamethylpentasiloxane and their mixtures.

The composition according to the invention can in particular comprise one or more silicone oils, generally in a small amount, that is to say an amount which can be less than 10% by weight of the solvent phase. The silicone oils can in particular be volatile or nonvolatile oils, such as dimethicone, phenyl dimethicone, alkyl dimethicone, dimethicone copolyol or cyclomethicone.

The volatile oil can be present in the composition according to the invention at a content ranging from 0.1 to 98% by weight, in particular from 1 to 65% by weight and especially from 2 to 50% by weight, with respect to the total weight of the composition.

The nonvolatile oils can in particular be chosen from nonvolatile hydrocarbon oils, if appropriate fluorinated, and/or nonvolatile silicone oils.

Mention may in particular be made, as nonvolatile hydrocarbon oil, of:

- hydrocarbon oils of animal origin,
- hydrocarbon oils of vegetable origin, such as triglycerides composed of esters of fatty acids and of glycerol, the fatty acids of which can have various chain lengths from $C_4$ to $C_{24}$, it being possible for these chains to be linear or branched and saturated or unsaturated; these oils are in particular wheat germ, sunflower, grape seed, sesame, maize, apricot, castor, shea, avocado, olive, soybean, sweet almond, palm, rapeseed, cottonseed, hazelnut, macadamia, jojoba, alfalfa, poppy, pumpkinseed, sesame, cucumber, blackcurrant seed, evening primrose, millet, barley,

quinoa, rye, safflower, candlenut, passionflower or musk rose oil; shea butter; or triglycerides of caprylic/capric acids, such as those sold by Stéarineries Dubois or those sold under the names Miglyol 810®, 812®, and 818® by Dynamit Nobel;

- synthetic ethers having from 10 to 40 carbon atoms;
- linear or branched hydrocarbons of mineral or synthetic origin, such as liquid petrolatum, polydecenes, hydrogenated polyisobutene, such as parleam, squalane, and their mixtures;
- synthetic esters, such as oils of formula $R^1COOR^2$ in which $R^1$ represents the residue of a linear or branched fatty acid comprising from 1 to 40 carbon atoms and $R^2$ represents a hydrocarbon chain, in particular a branched hydrocarbon chain, comprising from 1 to 40 carbon atoms, provided that $R^1 + R^2$ is ≥ 10, such as, for example, Purcellin oil (cetearyl octanoate), isopropyl myristate, isopropyl palmitate, $C_{12}$ to $C_{15}$ alkyl benzoates, hexyl laurate, diisopropyl adipate, isononyl isononanoate, 2-ethylhexyl palmitate, isostearyl isostearate, or heptanoates, octanoates, decanoates or ricinoates of alcohols or of polyalcohols, such as propylene glycol dioctanoate; hydroxylated esters, such as isostearyl lactate or diisostearyl malate; esters of polyols and esters of pentaerythritol;
- fatty alcohols which are liquid at ambient temperature with a branched and/or unsaturated carbon chain having from 12 to 26 carbon atoms, such as octyldodecanol, isostearyl alcohol, oleyl alcohol, 2-hexyldecanol, 2-butyloctanol and 2-undecylpentadecanol;
- higher fatty acids, such as oleic acid, linoleic acid, linolenic acid and their mixtures.

The nonvolatile silicone oils which can be used in the composition according to the invention can be nonvolatile polydimethylsiloxanes (PDMSs), polydimethylsiloxanes comprising pendant alkyl or alkoxy groups and/or alkyl or alkoxy groups at the end of the silicone chain, which groups each have from 2 to 24 carbon atoms, or phenylated silicones, such as phenyl trimethicones, phenyl dimethicones, phenyl(trimethylsiloxy)diphenylsiloxanes, diphenyl dimethicones, diphenyl(methyldiphenyl)trisiloxanes and (2-phenylethyl)trimethylsiloxysilicates.

The nonvolatile oils can be present in the composition according to the invention in a content ranging from 0.01% to 90% by weight, in particular from 0.1% to 85% by weight and especially from 1% to 70% by weight, with respect to the total weight of the composition.

More generally, the fatty substance which is liquid at ambient temperature and at atmospheric pressure can be present in a proportion of 0.01 to 90% by weight and in particular from 0.1 to 85% by weight, with respect to the weight of the fatty phase.

As regards the fatty substance which is solid at ambient temperature and at atmospheric pressure, it can be chosen from waxes, pasty fatty substances, gums and their mixtures. This solid fatty substance can be present in a proportion of 0.01 to 50% by weight, in particular of 0.1 to 40% by weight and especially of 0.2 to 30% by weight, with respect to the total weight of the fatty phase.

Thus, the composition according to the invention can comprise at least one fatty compound which is pasty at ambient temperature.

The term "pasty fatty substance" is understood to mean, within the meaning of the invention, fatty substances having a melting point ranging from 20 to 55°C, preferably 25 to 45°C, and/or a viscosity at 40°C ranging from 0.1 to 40 Pa·s (1 to 400 poises), in particular 0.5 to 25 Pa·s, measured with a Contraves TV or Rheomat 80 equipped with a spindle rotating at 60 Hz. A person skilled in the art can choose the spindle which makes it possible to measure the viscosity from the MS-r3 and MS-r4 spindles on the basis of his general knowledge, so as to be able to carry out the measurement of the pasty compound tested.

More particularly, these fatty substances can be hydrocarbon compounds, optionally of polymeric type; they can also be chosen from silicone compounds; they can also be provided in the form of a mixture of hydrocarbon and/or silicone compounds. In the case of a mixture of various pasty fatty substances, use is preferably made of predominantly hydrocarbon pasty compounds (comprising mainly carbon and hydrogen atoms and optionally ester groups).

Mention may be made, among pasty compounds capable of being used in the composition according to the invention, of lanolins and lanolin derivatives, such as acetylated lanolins, oxypropylenated lanolins or isopropyl lanolate, having a viscosity of 18 to 21 Pa·s, preferably 19 to 20.5 Pa·s, and/or a melting point of 30 to 55°C, and their mixtures. Use may also be made of esters of fatty acids or fatty alcohols, in particular those having 20 to 65 carbon atoms (melting point of the order of 20 to 35°C and/or viscosity at 40°C ranging from 0.1 to 40 Pa·s), such as triisostearyl or cetyl citrate; arachidyl propionate; poly(vinyl laurate); cholesterol esters, such as triglycerides of vegetable origin, such as hydrogenated vegetable oils, viscous polyesters, and their mixtures. Use may be made, as triglyceride of vegetable origin, of derivatives of hydrogenated castor oil, such as "Thixinr®" from Rheox.

Mention may also be made of silicone pasty fatty substances, such as polydimethylsiloxanes (PDMSs) with high molecular weights and in particular those having pendant chains of the alkyl or alkoxy type having from 8 to 24 carbon atoms and a melting point of from 20-55°C, such as stearyl dimethicones, in particular those sold by Dow Corning under the trade names of DC2503® and DC25514®, and their mixtures.

The pasty fatty substance can be present in the composition according to the invention in a content ranging

from 0.01 to 50% by weight, in particular ranging from 0.1 to 45% by weight and especially ranging from 0.2 to 30% by weight, with respect to the total weight of the composition.

The composition according to the invention can additionally comprise a wax. The wax can be solid at ambient temperature (25°C), with a reversible solid/liquid change of state, having a melting point of greater than 30°C which can range up to 200°C, having a hardness of greater than 0.5 MPa and exhibiting, in the solid state, an anisotropic crystalline organization. It can be a hydrocarbon, fluorinated and/or silicone wax and can be of animal, vegetable, mineral or synthetic origin. It can be chosen, for example, from beeswax, carnauba wax, candelilla wax, paraffin waxes, hydrogenated castor oil, silicone waxes, microcrystalline waxes and their mixtures.

In particular, the wax can be present in the form of a wax(es)-in-water emulsion.

The wax can be present in the composition according to the invention in a content ranging from 0.01 to 50% by weight, in particular from 0.1 to 30% by weight and especially from 0.2 to 20% by weight, with respect to the total weight of the composition.

## SURFACE-ACTIVE AGENTS

The composition according to the invention can additionally comprise at least one surface-active agent present in particular in a proportion ranging from 0.1 to 30% by weight and better still from 5 to 15% by weight, with respect to the total weight of the composition.

This surface-active agent can be chosen from anionic, cationic, amphoteric or nonionic surface-active agents. Reference may be made to the document "Encyclopedia of Chemical Technology, Kirk-Othmer", volume 22, pp. 333-432, 3rd edition, 1979, Wiley for the definition of the (emulsifying) properties and functions of surfactants, in particular pp. 347-377 of this reference for anionic and nonionic surfactants.

The surfactants preferably used in the composition according to the invention are chosen from

- nonionic surfactants, such as fatty acids and fatty alcohols,
- and/or anionic surfactants.

## PARTICULATE PHASE

The composition of the invention can furthermore comprise an additional particulate phase which can be present in a proportion of 0.01 to 40% by weight, in particular of 0.01 to 30% by weight and especially of 0.05 to 20% by weight, with respect to the total weight of the composition.

It can in particular comprise at least one pigment and/or at least one pearlescent agent and/or at least one additional filler used in cosmetic compositions.

The term "pigments" should be understood as comprising white or colored and inorganic or organic particles which are insoluble in the liquid hydrophilic phase and which are intended to color and/or opacify the composition. The term "fillers" should be understood as comprising colorless or white, inorganic or synthetic and lamellar or nonlamellar particles. The term "pearlescent agents" should be understood as comprising iridescent particles, in particular produced by certain shellfish in their shells or else synthesized.

The pigments can be present in the composition in a proportion of 0.01 to 25% by weight, in particular of 0.01 to 15% by weight and especially of 0.02 to 5% by weight, with respect to the total weight of the composition.

Mention may be made, as inorganic pigments which can be used in the invention, of titanium, zirconium or cerium oxides, zinc, iron or chromium oxides, ferric blue, manganese violet, ultramarine blue and chromium hydrate. Mention may be made, among organic pigments which can be used in the invention, of carbon black, pigments of D & C type, lakes based on cochineal carmine, barium, strontium, calcium or aluminum, or the diketopyrrolopyrroles (DPP) disclosed in the documents EP-A-542 669, EP-A-787 730, EP-A-787 731 and WO-A-96/08537. The amount and/or the choice of these pigments are generally adjusted by taking into account the amount of rod-coil polymers present in the cosmetic composition under consideration.

The pearlescent agents can be present in the composition in a proportion of 0.01 to 25% by weight, in particular of 0.01 to 15% by weight and especially of 0.02 to 5% by weight, with respect to the total weight of the composition.

The pearlescent pigments can be chosen from white pearlescent pigments, such as mica covered with titanium oxide or bismuth oxychloride, colored pearlescent pigments, such as titanium oxide-coated mica with iron oxides, titanium oxide-coated mica with in particular ferric blue or chromium oxide, or titanium oxide-coated mica with an organic pigment of the abovementioned type, and pearlescent pigments based on bismuth oxychloride.

The additional fillers can be present in a proportion of 0.01 to 40% by weight, in particular of 0.01 to 30% by weight and especially of 0.02 to 20% by weight, with respect to the total weight of the composition. Their amounts are also generally adjusted by taking into account the amount of rod-coil polymers.

They can in particular be spherical fillers, such as, for example, talc, zinc stearate, mica, kaolin, polyamide

(Nylon®) powders (Orgasol® from Atochem), polyethylene powders, tetrafluoroethylene polymer (Teflon®) powders, starch, boron nitride, polymer microspheres, such as those of poly(vinylidene chloride)/acrylonitrile, for example Expancel® (Nobel Industrie), or of acrylic acid copolymers (Polytrap® from Dow Corning), silicone resin microbeads (Tospearls® from Toshiba, for example) and organopolysiloxane elastomers.

The composition can also comprise water-soluble or fat-soluble dyes in a content ranging from 0.01 to 6% by weight with respect to the total weight of the composition, in particular ranging from 0.01 to 3% by weight. The fat-soluble dyes are, for example, Sudan red, DC Red 17, DC Green 6, β-carotene, soybean oil, Sudan brown, DC Yellow 11, DC Violet 2, DC Orange 5 and quinoline yellow. The water-soluble dyes are, for example, beetroot juice and methylene blue.

The composition according to the invention can additionally comprise any ingredient conventionally used in the fields under consideration and more especially in the cosmetics and dermatological fields. These ingredients are chosen in particular from vitamins, antioxidants, thickening agents, trace elements, softening agents, sequestering agents, fragrances, basifying or acidifying agents, preservatives, UV screening agents, hydrophilic or lipophilic active principles, and their mixtures. The amounts of these various ingredients are those conventionally used in the fields under consideration and are, for example, from 0.01 to 20% of the total weight of the composition.

Of course, a person skilled in the art will take care to choose this or these optional additional compound or compounds and/or their amounts so that the advantageous properties of the composition according to the invention are not, or not substantially, detrimentally affected by the envisaged addition.

The composition of the invention can be obtained according to the preparation processes conventionally used in cosmetics or in dermatology.

It can be provided in the form of an oily solution or of a direct or inverse emulsion.

It can also be provided in the form of a product cast as a stick or in a dish, such as lipsticks or lip balms, cast foundations, concealers, products for "correcting" and/or "embellishing" the complexion, eye shadows and face powders.

It can be provided in the form of a product for caring for and/or making up the nails, skin and/or lips.

It can also be provided in the form of a hair product for caring for and/or styling the hair, whether for lacquers, sprays, gels, mousses, shampoos and conditioners.

It can be a formulation in the presence of a propellent, which can be any liquefiable gas commonly used in aerosol devices.

The composition examples below are given by way of illustration and without a limiting nature.

Example 1:

Synthesis of a block copolymer of poly(ethylene oxide)-b-poly(aromatic ester) type, as follows:

Ethyl 4-[4-[methoxypoly(ethyleneoxy)ethyloxy]-4'-biphenylcarbonyloxy]-4'-biphenylcarboxylate.

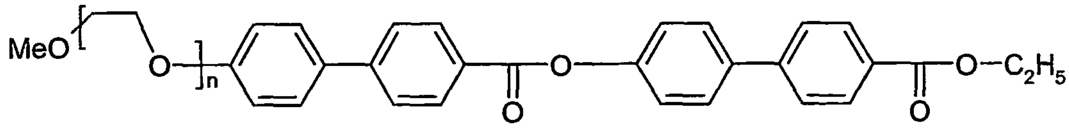

*Stage 1: Tosylation of poly(ethylene glycol) 350 methyl ether [PEG 350 OMe].*

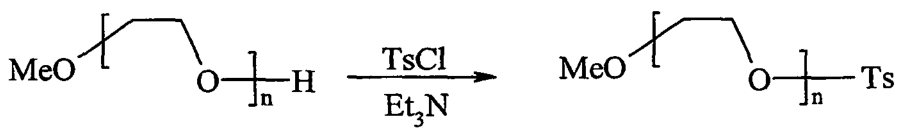


1 2

The following are introduced into a 100 ml three-necked flask equipped with a 50 ml dropping funnel:

- 5 g of PEG 350 OMe (14.3 mmol, 1 eq.),

- 4.84 g of triethylamine (48 mmol, 3.35 eq.) in 15 ml of dichloromethane.

The reaction medium, kept under argon, is stirred and maintained at 0°C with an ice bath.

A mixture of 6.74 g of para-toluenesulfonyl chloride (TsCl) (37.7 mmol) in 17 ml of dichloromethane is added dropwise to the reaction medium over approximately 30 minutes.

The reaction medium is maintained at 0°C for 4 h after the end of the addition. The mixture, initially homogeneous, gradually becomes white-opaque with the appearance of a precipitate (triethylamine salt).

Purification:

The product is filtered three times through a sintered glass funnel (porosity 4) in order to remove the triethylamine salt formed. The filtrate is precipitated from cold diethyl ether (ratio by volume 1/10) and the ethereal phase and the precipitate are respectively evaporated and dried on a rotary evaporator.

An NMR analysis of the two fractions shows that the tosylated polymer is present in the ethereal phase and that the precipitate predominantly comprises triethylamine salt. The precipitate is redissolved in dichloromethane for a further precipitation in order to recover the maximum of product.

The evaporation of the ethereal phases results in an oil being obtained, which oil is washed with petroleum ether until the para-toluenesulfonyl chloride has disappeared, monitored by $^1H$ NMR.

The tosylated polymer 2 is a yellow/brown oil characterized by $^1H$ NMR.

Characterization:

Compound 1: $^1H$ NMR (400 MHz, $CDCl_3$, δ, ppm): 3.37 (s, 3H, $OCH_3$), 3.53-3.71 (m, 30H, $CH_2CH_2O$)

Compound 2: $^1H$ NMR (400 MHz, $CDCl_3$, δ, ppm): 2.25 (s, 3H, $CH_3$-∅), 3.28 (s, 3H, $CH_3O$), 4.44-4.08 (m, 31H, $CH_2CH_2O$), 7.23 (d, 2H, Ar-H, J = 8.2 Hz), 7.69 (d, 2H, Ar-H, J = 10.4 Hz).

*Stage 2: Coupling with ethyl 4'-hydroxy-4-biphenylcarboxylate*

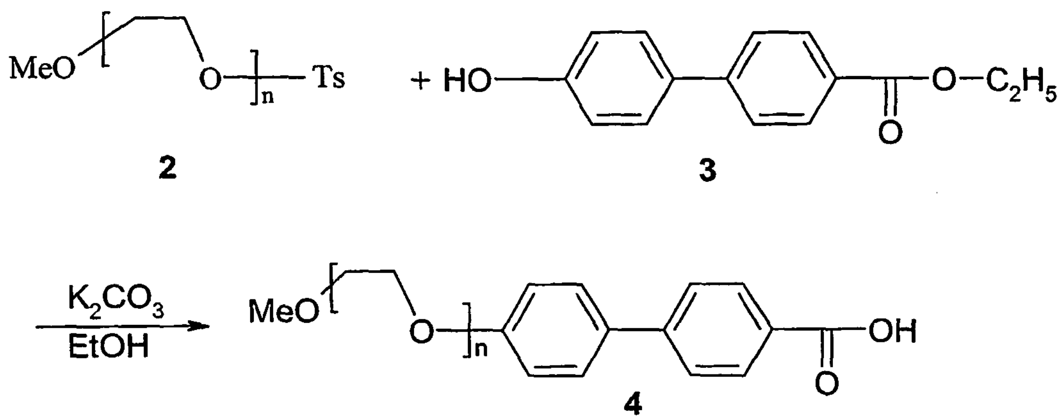


2

3

4

The following are introduced into a 250 ml round-bottomed flask:

- 2 g of compound 2 (3.97 mmol, 1 eq.),
  2.88 g of ethyl 4'-hydroxy-4-biphenylcarboxylate, compound 3 (11.9 mmol, 3 eq.),
- 2.76 g of potassium carbonate (19.8 mmol) in 87.8 ml of synthetic ethanol.

The yellow mixture is brought to reflux for 24 h and a white precipitate appears during the reaction.

Hydrolysis of the ester functional groups:

The hydrolysis is carried out in situ on the nonisolated product.

After cooling, a mixture of 3.34 g of KOH in 20 ml of distilled water is added dropwise to the preceding mixture and is left stirring at ambient temperature for 3 hours.

Purification:

The product is dissolved in water and extracted 3 times with dichloromethane.

The dichloromethane phases are washed with water, dried over sodium sulfate and filtered before being evaporated on a rotary evaporator.

To confirm that there is no more free alcohol 3, an investigation is carried out by TLC with ethyl acetate as eluent, Rf = 0.75.

The product 4 is a viscous white solid characterized by $^1H$ NMR.

Characterization:

Compound 3: $^1H$ NMR (400 MHz, $CDCl_3$, 5, ppm): 1.42 (t, $CH_3CH_2O$, J = 7.12 Hz), 4.4 (q, 2H, $CH_3CH_2O$, J = 7.14 Hz), 6.9 (d, 2H, Ar-H, J = 8.76 Hz), 7.5 (d, 2H, Ar-H, J = 8.76 Hz), 7.6 (d, 2H, Ar-H, J = 8.4 Hz), 8.1 (d, 2H, Ar-H, J = 8.4 Hz).

Compound 4: $^1H$ NMR (400 MHz, $CDCl_3$, 5, ppm): 3.33 (s, 3H, $CH_3O$), 3.36-1.41 (m, 35H, $CH_2CH_2O$), 6.92 (d, 2H, Ar-H, J = 8.4 Hz), 7.57 (d, 2H, Ar-H, J = 8.4 Hz), 7.48 (d, 2H, Ar-H, J = 8.6 Hz), 6.9 (d, 2H, Ar-H, J = 8.6 Hz).

*Stage 3: Esterification with ethyl 4'-hydroxy-4-biphenylcarboxylate*

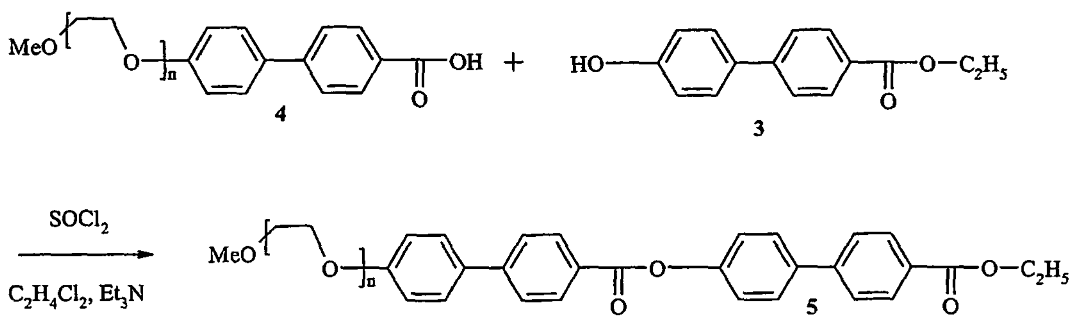


4 3 5

The following are introduced into a 50 ml round-bottomed flask:

- 0.5 g of compound 4 (0.983 mmol, 1 eq.) in 5 ml of 1,2-dichloroethane,
- 134 µl of thionyl chloride (1.85 mmol, 2 eq.), added dropwise.

The mixture is brought to reflux for 4 hours. Evolution of hydrochloric acid is observed at the top of the reflux condenser using pH paper.

After cooling to ambient temperature, a mixture of 0.46 g of ethyl 4'-hydroxy-4-biphenylcarboxylate, compound 3 (1.96 mmol, 2 eq.), dissolved in 2 ml of triethylamine (HCl scavenger), is added dropwise.

The reaction medium is left at 40°C for 1 h and is then brought to reflux for 2 h.

The final mixture obtained in dichloroethane is brown.

Purification:

The product is precipitated from methanol.

The methanol phase and the precipitate are respectively evaporated and dried on a rotary evaporator.

A second precipitation of the filtrate may be necessary if a portion of the product has dissolved in the methanol.

As described in stage 2, an investigation by TLC (eluent = ethyl acetate) is carried out to confirm that free alcohol 3 does not remain in the medium, Rf = 0.75.

The final product 5 is a brown paste which can be dispersed in water. It is characterized by $^1H$ NMR.

Compound 5: $^1H$ NMR (400 MHz, $CDCl_3$, $\delta$, ppm): 1.33 (t, 4H, $CH_3CH_2O$, J = 6.98 Hz), 3.27 (s, 3H, $CH_3O$), 3.44-4.32 (m, 39H, $CH_2CH_2O$), 4.30 (q, 2. 8H, $CH_3CH_2O$), 6.9 (d, 2H, Ar-H, J = 8.8 Hz), 7.25 (d, 2H, Ar-H, J = 8.76 Hz), 7.5 (d, 2H, Ar-H, J = 8.76 Hz), 7.6 (m, 6H, Ar-H), 8.03 (d, 2H, Ar-H, J = 8.24 Hz), 8.16 (d, 2H, Ar-H, J = 8.4 Hz).

Example 2:

Preparation of a hair composition according to the invention

The copolymer obtained in example 1 above is dissolved in a proportion of 10% by weight of the total weight of the composition in an aqueous solution in the presence of 0.5% by weight of Parabens® as preservative. It is applied to the hair using a pump-action spray.

## Claims

1. A cosmetic composition comprising, in a physiologically acceptable medium:

   - at least one aqueous phase,
   - at least one preservative, and
   - at least one block copolymer of rod-coil type comprising at least one coil polymeric block structure with a variable conformation covalently bonded to at least one rod polymeric block structure with a restricted conformation,

   said block copolymer of rod-coil type being employed at concentrations capable of conferring, on the physiologically acceptable medium comprising it, a viscosity suited to the desired formulation.

2. The cosmetic composition as claimed in claim 1, **characterized in that** said rod polymeric structure with a restricted conformation incorporates, in the chemical structure, at least one and in particular at least two aromatic ring systems.

3. The cosmetic composition as claimed in claim 1 or 2, **characterized in that** the restricted conformation of the rod polymeric structure is provided, in all or part, by $\pi$-$\pi$ interactions.

4. The cosmetic composition as claimed in any one of claims 1 to 3, **characterized in that** the mean distance between the chain ends in the coil block, namely $\langle R_0^2 \rangle_{coil}$, satisfies the convention $\langle R_0^2 \rangle_{coil} = NL^2$, where

   L represents the length of a monomer, and
   N represents the number of monomers constituting the block.

5. The cosmetic composition as claimed in any one of claims 1 to 4, **characterized in that** the coil block is composed of one or more copolymers or homopolymers deriving from the radical polymerization of monomers comprising ethylene, vinyl, allyl, (meth)acrylate and/or (meth)acrylamide units and derivatives.

6. The cosmetic composition as claimed in any one of claims 1 to 5, **characterized in that** the coil polymer is chosen from:

   - vinyl/(meth)acrylate, vinyl/(meth)acrylamide, vinyl/(meth)acrylate/methacrylamide, olefin/vinyl and (meth)acrylate/(meth)acrylamide copolymers,
   - homopolymers or copolymers based on vinyl acetate, on styrene, on vinylpyrrolidone, on vinylcaprolactam, on poly(ethylene oxide) (meth)acrylate, on stearyl (meth)acrylate, on lauryl (meth)acrylate, on vinyllaurate, on butyl (meth)acrylate, on ethylhexyl (meth)acrylate, on crotonic acid, on (meth)acrylic acid, on maleic anhydride, on styrenesulfonic acid, on dimethyldiallylamine, on vinylpyridine, on dimethylaminoethyl (meth)acrylate and on dimethylaminopropyl(meth)acrylamide,
   - polycondensates of polyurethane and/or polyurea type, aliphatic polyesters, aliphatic polyamides or their copolymers, such as, for example, polyurethane/urea and polyester/amide,
   - polymers obtained by ring opening, such as polyethers of poly(ethylene oxide) or poly(propylene oxide) type and their poly(ethylene oxide)/poly(propylene oxide) copolymers; polyesters, such as polycaprolactone; and polyoxazolines, such as poly(2-methyloxazoline) or poly(2-ethyloxazoline),
   - siloxane (co)polymers, such as polydimethylsiloxane (PDMS) and polymethylphenylsiloxane,
   - polymers obtained by metathesis, such as poly(norbornene) and its copolymers,
   - polymers obtained by cationic polymerization, such as poly(vinyl alkyl ether)s,
   - copolymers of these various types of polymer, such as, for example, polysiloxane/poly(ethylene oxide)s, and
   - their salts and derivatives.

**7.** The cosmetic composition as claimed in any one of claims 1 to 6, **characterized in that** the number mass of the coil blocks varies from 500 g/mol to 1 000 000 g/mol, in particular from 500 g/mol to 800 000 g/mol, and more particularly from 500 g/mol to 500 000 g/mol.

**8.** The cosmetic composition as claimed in any one of claims 1 to 7, **characterized in that** the rod polymeric structure exhibits a mean distance between the chain ends, $<R_0^2>_{rod}$, which satisfies the convention:

$$<R_0^2>_{rod} = CNL^2,$$

where

L represents the length of a monomer,
C represents the restrictions imposed on the chain, with C greater than 1 and in particular varying from 4 to 10, and
N represents the number of monomers constituting the block.

**9.** The cosmetic composition as claimed in any one of claims 1 to 8, **characterized in that** the rod block comprises at least one aromatic structure of formula:

$$-[Ar]-_n$$

with:

- it being possible for n to vary from 2 to 1000, in particular from 5 to 700 and especially from 20 to 700, and
- it being possible for the Ar group(s) to represent one or more aromatic unit(s) or derivative(s) of aromatic units,

the combined aromatic units constituting said formula being identical or different in chemical nature.

**10.** The cosmetic composition as claimed in any one of claims 1 to 9, **characterized in that** the rod block is chosen from:

- polyarylenes, poly(arylene vinylene) s, poly(arylene ethynylene)s or poly(arylene ether)s and, among these, poly(para-phenylene)s, poly(para-phenylene vinylene)s, poly (para-phenylene ethynylene)s, poly(para-phenylene ether)s, poly(para-phenylene sulfide)s and poly(biphenylene ether)s,
- polyquinolines, polyquinoxalines, polypyridines, poly(pyridine vinylene)s, poly(naphthylene vinylene)s, polythiophenes, poly(thiophene vinylene)s, poly(thiophene ethynylene)s, polybisthiophenes, polyfurans, polypyrroles, polyanilines, polybenzimidazoles, polybenzothiazoles, polybenzoxazoles, polybenzobisazoles, aromatic polyamides, aromatic polyhydrazides, aromatic polyazomethines, aromatic polyesters, aromatic polyimides, polycarbazoles, polyfluorenes or polyanthracenes, and
- their copolymers.

**11.** The cosmetic composition as claimed in any one of claims 1 to 10, **characterized in that** the rod block is chosen from:

- poly[2,5-bis(4-methoxyphenyl)oxycarbonyl]styrene,
- poly(4'-oxybiphenyl),
- poly(phenylene benzobisthiazole),
- poly(1,4-(2,5-dihexylphenylene)),
- poly(1,4-(2,5-dihexylphenylene) ethynylene),
- poly(1,4-(2,5 dihexylphenylene) vinylene),
- poly(3-dodecylthiophene),
- poly(3-octylthiophene),
- poly[1,4-(2,5-di(polydimethylsiloxane)phenylene)], and
- their copolymers.

**12.** The cosmetic composition as claimed in any one of claims 1 to 11, **characterized in that** the rod block is water-soluble or water-dispersible.

**13.** The cosmetic composition as claimed in any one of claims 1 to 12, **characterized in that** the rod block has one or

more solubilizing groups capable of rendering it dispersible or soluble in an aqueous phase.

**14.** The cosmetic composition as claimed in claim 13, **characterized in that** the solubilizing group is chosen from the following units : -COOH, $-CH_2COOH$, $-(CH_2)_2COOH$, $-(CH_2)_3COOH$, -OH, $-CH_2OH$, $-(CH_2)_6OH$, $-(CH_2)SO_3H$, $-(CH_2)_2SO_3H$, - $(CH_2)_3SO_3H$, $-O(CH_2)_3SO_3H$, - $(CH_2)_2N(CH_3)(CH_3)$, - $N(CH_3)(CH_3)$, $-O(CH_2)_3N(CH_2CH_3)_2$, - $(CH_2)_2N(CH_2CH_3)_2$, - $[(CH_2)_2O]_xCH_2CH_2OH$, $-[(CH_2)_2O]_xCH_2CH_2OCH_3$ or $-[(CH_2)_2O]_xCH_2CH_2OCH_2CH_3$, with x symbolizing a mean number of between 0 and 200, and their sodium and amine salts, in particular their triethylamine or tributylamine salts.

**15.** The cosmetic composition as claimed in any one of claims 1 to 14, **characterized in that** the number mass of the rod blocks varies from 200 g/mol to 1 000 000 g/mol, in particular from 250 g/mol to 800 000 g/mol, and more particularly from 250 g/mol to 500 000 g/mol.

**16.** The cosmetic composition as claimed in any one of claims 1 to 15, **characterized in that** the rod blocks are present in a proportion of at least 10%, in particular of at least 15%, especially of at least 30%, and in particular in a proportion of at most 90%, especially of at most 85%, indeed even of at most 80%, by weight, with respect to the total weight of the copolymer.

**17.** The cosmetic composition as claimed in any one of claims 1 to 16, **characterized in that** the number overall mass of the rod-coil copolymer varies from 700 g/mol to 1 000 000 g/mol, in particular from 1000 g/mol to 800 000 g/mol and more particularly from 2000 g/mol to 500 000 g/mol.

**18.** The cosmetic composition as claimed in any one of claims 1 to 17, **characterized in that** it comprises at least one rod-coil block copolymer chosen from:

• diblocks: rod-b-coil

- polydimethylsiloxane-b-poly[2,5-bis(4-methoxyphenyl)-oxycarbonyl]styrene,
- poly(ethylene/butylene)-b-poly(4'-oxybiphenyl),
- alkyd polyester with $C_{12}$ carbon chains-b-poly(biphenyl-4'-biphenylcarboxylate),
- poly(vinyl laurate)-b-poly(phenylene benzobisthiazole),
- polyisoprene-b-poly(1,4-(2,5-dihexylphenylene)), and

• triblocks: coil-b-rod-b-coil

- polydimethylsiloxane-b-poly(1,4-(2,5-dihexylphenylene) ethynylene)-b-polydimethylsiloxane,
- poly(ethylene/butylene)-b-poly(1,4-(2,5-dihexylphenylene) ethynylene)-b-poly(ethylene/butylene),
- alkyd polyester with $C_{12}$ carbon chains-b-poly(biphenyl-4'-biphenylcarboxylate)-b-alkyd polyester with $C_{12}$ carbon chains,
- poly(vinyl laurate)-b-poly(4'-oxybiphenyl)-b-poly(vinyl laurate),
- poly(oleyl acrylate)-b-poly(2,5-(hexylthiophene))-b-poly(oleyl acrylate), and
- their salts or derivatives.

**19.** The cosmetic composition as claimed in any one of claims 1 to 18, **characterized in that** it comprises from 0.5 to 90% by weight, in particular from 0.7 to 85% by weight and more particularly from 0.8 to 75% by weight of rod-coil block copolymer(s), with respect to the total weight of the composition.

**20.** The cosmetic composition as claimed in any one of claims 1 to 19, **characterized in that** it comprises at least one preservative chosen from esters of para-hydroxybenzoic acid, phenoxyethanol, compounds which release formaldehyde, sodium benzoate, chlorhexidine digluconate, alkytrimethylammonium bromides and their mixtures, and polymer quaternary ammonium salts, such as polyquaternium-10, and their mixtures.

**21.** The cosmetic composition as claimed in any one of claims 1 to 20, **characterized in that** it comprises from 0.001 to 10% by weight of preservative, with respect to the total weight of the composition.

**22.** The cosmetic composition as claimed in any one of claims 1 to 21, **characterized in that** the aqueous phase comprises a mixture of water and of water-miscible organic solvent.

23. The cosmetic composition as claimed in any one of claims 1 to 22, **characterized in that** it comprises from 1 to 95% by weight of aqueous phase, with respect to the total weight of the composition.

24. The cosmetic composition as claimed in any one of claims 1 to 23, **characterized in that** it additionally comprises at least one fatty phase.

25. The cosmetic composition as claimed in claim 24, **characterized in that** said fatty phase comprises at least one fatty substance which is liquid at ambient temperature and at atmospheric pressure and/or at least one fatty substance which is solid at ambient temperature and at atmospheric pressure.

26. The cosmetic composition as claimed in claim 25, **characterized in that** said fatty substance which is liquid at ambient temperature and at atmospheric pressure comprises at least one volatile or nonvolatile oil or one of their mixtures.

27. The cosmetic composition as claimed in claim 26, **characterized in that** said at least one nonvolatile oil represents from 0.01 to 90% by weight, in particular from 0.1 to 85% by weight, with respect to the total weight of the fatty phase.

28. The cosmetic composition as claimed in any one of claims 25 to 27, **characterized in that** said fatty substance which is solid at ambient temperature and at atmospheric pressure is chosen from waxes, pasty fatty substances, gums and their mixtures.

29. The cosmetic composition as claimed in any one of claims 25 to 28, **characterized in that** said fatty phase comprises at least one solid fatty substance in a proportion of 0.01 to 50% by weight, in particular of 0.1 to 40% by weight and especially of 0.2 to 30% by weight, with respect to the total weight of the composition.

30. The cosmetic composition as claimed in any one of claims 1 to 29, **characterized in that** said composition additionally comprises a particulate phase in a proportion of 0.01 to 40% by weight, in particular of 0.01 to 30% by weight and especially of 0.05 to 20% by weight, with respect to the total weight of said composition.

31. The cosmetic composition as claimed in claim 30, **characterized in that** said particulate phase comprises at least one pigment and/or at least one pearlescent agent and/or at least one additional filler.

32. The cosmetic composition as claimed in any one of claims 1 to 31, **characterized in that** it is provided in the form of a direct or inverse emulsion.

33. The cosmetic composition as claimed in any one of claims 1 to 32, **characterized in that** it is provided in the form of products cast as a stick or in a dish.

34. The cosmetic composition as claimed in any one of claims 1 to 33, **characterized in that** it is provided in the form of a makeup and/or care product for the skin and/or the lips.

35. The cosmetic composition as claimed in any one of claims 1 to 31, **characterized in that** it is provided in the form of a product for caring for and/or making up the nails.

36. A cosmetic composition as claimed in any one of claims 1 to 31, **characterized in that** it is provided in the form of a product for caring for and/or styling the hair.

37. The use of a rod-coil copolymer as defined in any one of claims 1 to 19 as rheological agent in a cosmetic composition comprising at least one preservative and at least one aqueous phase.

38. A method for making up and/or caring for the skin, superficial body growths and/or hair comprising at least one stage of application of a cosmetic composition as defined in any one of claims 1 to 36.

## Patentansprüche

1. Kosmetische Zusammensetzung, umfassend in einem physiologisch verträglichen Medium:

- mindestens eine wässrige Phase,
- mindestens einen Konservierungsstoff und
- mindestens ein Blockcopolymer vom Stab-Knäuel-Typ, umfassend mindestens eine polymere Knäuel-Blockstruktur mit einer variablen Konformation kovalent gebunden an mindestens eine polymere Stab-Blockstruktur mit einer eingeschränkten Konformation,

wobei das Blockcopolymer vom Stab-Knäuel-Typ in Konzentrationen verwendet wird, welche zum Übertragen einer Viskosität, welche für die gewünschte Formulierung geeignet ist, auf das physiologisch verträgliche Medium, welches es umfasst, in der Lage sind.

**2.** Kosmetische Zusammensetzung wie in Anspruch 1 beansprucht, **dadurch gekennzeichnet, dass** die polymere Stabstruktur mit einer eingeschränkten Konformation in der chemischen Struktur mindestens ein und insbesondere mindestens zwei aromatische Ringsysteme umfasst.

**3.** Kosmetische Zusammensetzung wie in Anspruch 1 oder 2 beansprucht, **dadurch gekennzeichnet, dass** die eingeschränkte Konformation der polymeren Stabstruktur insgesamt oder teilweise durch $\pi$-$\pi$-Wechselwirkungen bereitgestellt wird.

**4.** Kosmetische Zusammensetzung wie in einem der Ansprüche 1 bis 3 beansprucht, **dadurch gekennzeichnet, dass** der mittlere Abstand zwischen den Kettenenden in dem Knäuelblock, nämlich $<R_0^2>_{Knäuel}$, die Bedingung $<R_0^2>_{Knäuel} = NL^2$ erfüllt, wobei

L die Länge eines Monomers darstellt und
N die Anzahl von Monomeren, aus welchen der Block aufgebaut ist, darstellt.

**5.** Kosmetische Zusammensetzung wie in einem der Ansprüche 1 bis 4 beansprucht, **dadurch gekennzeichnet, dass** der Knäuelblock zusammengesetzt ist aus ein oder mehr Copolymeren oder Homopolymeren, welche sich ableiten von der Radikalpolymerisation von Monomeren, umfassend Ethylen-, Vinyl-, Allyl-, (Meth)acrylat- und/oder (Meth) acrylamid-Einheiten und Derivate.

**6.** Kosmetische Zusammensetzung wie in einem der Ansprüche 1 bis 5 beansprucht, **dadurch gekennzeichnet, dass** das Knäuelpolymer ausgewählt ist aus:

- Vinyl/(Meth)acrylat-, Vinyl/(Meth)acrylamid-, Vinyl/(Meth)acrylat/Methacrylamid-, Olefin/Vinyl- und (Meth)acrylat/(Meth)acrylamid-Copolymeren,
- Homopolymeren oder Copolymeren, basierend auf Vinylacetat, auf Styrol, auf Vinylpyrrolidon, auf Vinylcaprolactam, auf Poly(ethylenoxid)(meth)acrylat, auf Stearyl(meth)acrylat, auf Lauryl(meth)acrylat, auf Vinyllaurat, auf Butyl(meth)-acrylat, auf Ethylhexyl(meth)acrylat, auf Crotonsäure, auf (Meth)acrylsäure, auf Maleinsäureanhydrid, auf Styrolsulfonsäure, auf Dimethyldiallylamin, auf Vinylpyridin, auf Dimethylaminoethyl(meth)acrylat und auf Dimethylaminopropyl(meth)acrylamid,
- Polykondensaten vom Polyurethan- und/oder Polyharnstoff-Typ, aliphatischen Polyestern, aliphatischen Polyamiden oder ihren Copolymeren, wie zum Beispiel Polyurethan/harnstoff und Polyester/amid,
- durch Ringöffnung erhaltenen Polymeren, wie Polyether vom Poly(ethylenoxid)- oder Poly(propylenoxid)-Typ und ihren Poly(ethylenoxid)/Poly(propylenoxid)-Copolymeren; Polyestern, wie Polycaprolacton; und Polyoxazolinen, wie Poly(2-methyloxazolin) oder Poly(2-ethyloxazolin),
- Siloxan(co)polymeren, wie Polydimethylsiloxan (PDMS) und Polymethylphenylsiloxan,
- durch Metathese erhaltenen Polymeren, wie Poly(norbornen) und seinen Copolymeren,
- durch kationische Polymerisation erhaltenen Polymeren, wie Poly(vinylalkylether),
- Copolymeren dieser verschiedenen Polymertypen, wie zum Beispiel Polysiloxan/Poly(ethylenoxid)en und
- ihren Salzen und Derivaten.

**7.** Kosmetische Zusammensetzung wie in einem der Ansprüche 1 bis 6 beansprucht, **dadurch gekennzeichnet, dass** die Massenzahl der Knäuelblöcke von 500 g/mol bis 1 000 000 g/mol, insbesondere von 500 g/mol bis 800 000 g/mol und stärker besonders von 500 g/mol bis 500 000 g/mol variiert.

**8.** Kosmetische Zusammensetzung wie in einem der Ansprüche 1 bis 7 beansprucht, **dadurch gekennzeichnet, dass** die polymere Stabstruktur einen mittleren Abstand zwischen den Kettenenden, $<R_0^2>_{Stab}$, aufweist, welcher die folgende Bedingung erfüllt:

$$<R_0^2>_{Stab} = CNL^2,$$

wobei

L die Länge eines Monomers darstellt,
C die Einschränkungen, welche der Kette auferlegt sind, darstellt, wobei C höher als 1 ist und insbesondere von 4 bis 10 variiert, und
N die Anzahl von Monomeren, aus welchen der Block aufgebaut ist, darstellt.

**9.** Kosmetische Zusammensetzung wie in einem der Ansprüche 1 bis 8 beansprucht, **dadurch gekennzeichnet, dass** der Stabblock mindestens eine aromatische Struktur der folgenden Formel umfasst:

$$-[Ar]-_n$$

wobei:

- es für n möglich ist, von 2 bis 1000, insbesondere von 5 bis 700 und insbesondere von 20 bis 700 zu variieren, und
- es für die Ar-Rest(e) möglich ist, ein oder mehr aromatische Einheit(en) oder Derivat(e) von aromatischen Einheiten darzustellen,

wobei die kombinierten aromatischen Einheiten, aus welchen die Formel aufgebaut ist, identisch oder unterschiedlich in der chemischen Natur sind.

**10.** Kosmetische Zusammensetzung wie in einem der Ansprüche 1 bis 9 beansprucht, **dadurch gekennzeichnet, dass** der Stabblock ausgewählt ist aus:

- Polyarylenen, Poly(arylenvinylen)en, Poly(arylenethinylen)en oder Poly(arylenether)n und unter diesen Poly (para-phenylen)en, Poly(para-phenylenvinylen)en, Poly(para-phenylenethinylen)en, Poly(para-phenylenether) n, Poly(para-phenylensulfid)en und Poly(biphenylenether)n,
- Polychinolinen, Polychinoxalinen, Polypyridinen, Poly(pyridinvinylen)en, Poly(naphthylenvinylen)en, Polythiophenen, Poly(thiophenvinylen)en, Poly(thiophenethinylen)en, Polybisthiophenen, Polyfuranen, Polypyrrolen, Polyanilinen, Polybenzimidazolen, Polybenzothiazolen, Polybenzoxazolen, Polybenzobisazolen, aromatischen Polyamiden, aromatischen Polyhydraziden, aromatischen Polyazomethinen, aromatischen Polyestern, aromatischen Polyimiden, Polycarbazolen, Polyfluorenen oder Polyanthracenen, und
- ihren Copolymeren.

**11.** Kosmetische Zusammensetzung wie in einem der Ansprüche 1 bis 10 beansprucht, **dadurch gekennzeichnet, dass** der Stabblock ausgewählt ist aus:

- Poly[2,5-bis(4-methoxyphenyl)oxycarbonyl]styrol,
- Poly(4'-oxybiphenyl),
- Poly(phenylenbenzobisthiazol),
- Poly(1,4-(2,5-dihexylphenylen)),
- Poly(1,4-(2,5-dihexylphenylen)ethinylen),
- Poly(1,4-(2,5-dihexylphenylen)vinylen),
- Poly(3-dodecylthiophen),
- Poly(3-octylthiophen),
- Poly[1,4-(2,5-di(polydimethylsiloxan)phenylen)], und
- ihren Copolymeren.

**12.** Kosmetische Zusammensetzung wie in einem der Ansprüche 1 bis 11 beansprucht, **dadurch gekennzeichnet, dass** der Stabblock wasserlöslich oder wasserdispergierbar ist.

**13.** Kosmetische Zusammensetzung wie in einem der Ansprüche 1 bis 12 beansprucht, **dadurch gekennzeichnet, dass** der Stabblock einen oder mehr löslichkeitsvermittelnde Reste, welche zum Dispergierbarmachen oder Löslichmachen davon in einer wässrigen Phase in der Lage sind, aufweist.

**14.** Kosmetische Zusammensetzung wie in Anspruch 13 beansprucht, **dadurch gekennzeichnet, dass** der löslichkeitsvermittelnde Rest ausgewählt ist aus den folgenden Einheiten: -COOH, $-CH_2COOH$, $-(CH_2)_2COOH$, $-(CH_2)_3COOH$, -OH, $-CH_2OH$, - $(CH_2)_6OH$, $-(CH_2)SO_3H$, $-(CH_2)_2SO_3H$, $-(CH_2)_3SO_3H$, $-O(CH_2)_3SO_3H$, $-(CH_2)_2N-(CH_3)(CH_3)$, $-N(CH_3)(CH_3)$, $-O(CH_2)_3N(CH_2CH_3)_2$, $-(CH_2)_2N(CH_2CH_3)_2$, $-[(CH_2)_2-O]_xCH_2CH_2OH$, $-[(CH_2)_2O]_xCH_2CH_2OCH_3$ oder $-[(CH_2)_2O]_xCH_2CH_2OCH_2CH_3$, wobei x eine mittlere Anzahl von zwischen 0 und 200 darstellt, und ihren Natrium- und Aminsalzen, insbesondere ihren Triethylamin- oder Tributylaminsalzen.

**15.** Kosmetische Zusammensetzung wie in einem der Ansprüche 1 bis 14 beansprucht, **dadurch gekennzeichnet, dass** die Massenzahl der Stabblöcke von 200 g/mol bis 1 000 000 g/mol, insbesondere von 250 g/mol bis 800 000 g/mol und stärker besonders von 250 g/mol bis 500 000 g/mol variiert.

**16.** Kosmetische Zusammensetzung wie in einem der Ansprüche 1 bis 15 beansprucht, **dadurch gekennzeichnet, dass** die Stabblöcke in einem Anteil von mindestens 10 Gew.-%, insbesondere von mindestens 15 Gew.-%, insbesondere von mindestens 30 Gew.-%, und insbesondere in einem Anteil von höchstens 90 Gew.-%, insbesondere von höchstens 85 Gew.-%, in der Tat sogar von höchstens 80 Gew.-%, bezogen auf das Gesamtgewicht des Copolymers, vorhanden sind.

**17.** Kosmetische Zusammensetzung wie in einem der Ansprüche 1 bis 16 beansprucht, **dadurch gekennzeichnet, dass** die Gesamtmassenzahl des Stab-Knäuel-Copolymers von 700 g/mol bis 1 000 000 g/mol, insbesondere von 1000 g/mol bis 800 000 g/mol und stärker besonders von 2000 g/mol bis 500 000 g/mol variiert.

**18.** Kosmetische Zusammensetzung wie in einem der Ansprüche 1 bis 17 beansprucht, **dadurch gekennzeichnet, dass** sie mindestens ein Stab-Knäuel-Blockcopolymer umfasst, welches ausgewählt ist aus:

- Diblöcken: Stab-b-Knäuel

  - Polydimethylsiloxan-b-poly[2,5-bis(4-methoxyphenyl)oxycarbonyl]styrol,
  - Poly(ethylenlbutylen)-b-poly(4'-oxybiphenyl),
  - Alkydpolyester mit $C_{12}$-Kohlenstoffketten-b-poly(biphenyl-4'-biphenylcarboxylat),
  - Poly(vinyllaurat)-b-poly(phenylenbenzobisthiazol),
  - Polyisopren-b-poly(1,4-(2,5-dihexylphenylen)), und

- Triblöcken: Knäuel-b-Stab-b-Knäuel

  - Polydimethylsiloxan-b-poly(1,4-(2,5-dihexylphenylen)ethinylen)-b-polydimethylsiloxan,
  - Poly(ethylen/butylen)-b-poly(1,4-(2,5-dihexylphenylen)ethinylen)-b-poly-(ethylen/butylen),
  - Alkydpolyester mit $C_{12}$-Kohlenstoffketten-b-poly(biphenyl-4'-biphenylcarboxylat)-b-alkydpolyester mit $C_{12}$-Kohlenstoffketten,
  - Poly(vinyllaurat)-b-poly(4'-oxybiphenyl)-b-poly(vinyllaurat),
  - Poly(oleylacrylat)-b-poly(2,5-(hexylthiophen))-b-poly(oleylacrylat), und
  - ihren Salzen oder Derivaten.

**19.** Kosmetische Zusammensetzung wie in einem der Ansprüche 1 bis 18 beansprucht, **dadurch gekennzeichnet, dass** sie 0,5 bis 90 Gew.-%, insbesondere 0,7 bis 85 Gew.-% und stärker besonders 0,8 bis 75 Gew.-% Stab-Knäuel-Blockcopolymer(e), bezogen auf das Gesamtgewicht der Zusammensetzung, umfasst.

**20.** Kosmetische Zusammensetzung wie in einem der Ansprüche 1 bis 19 beansprucht, **dadurch gekennzeichnet, dass** sie mindestens einen Konservierungsstoff, ausgewählt aus Estern von para-Hydroxybenzoesäure, Phenoxyethanol, Verbindungen, welche Formaldehyd freisetzen, Natriumbenzoat, Chlorhexidindigluconat, Alkyltrimethylammoniumbromiden und ihren Gemischen, und quartären Ammoniumpolymersalzen, wie Polyquaternium-10, und ihren Gemischen, umfasst.

**21.** Kosmetische Zusammensetzung wie in einem der Ansprüche 1 bis 20 beansprucht, **dadurch gekennzeichnet, dass** sie 0,001 bis 10 Gew.-% Konservierungsstoff, bezogen auf das Gesamtgewicht der Zusammensetzung, umfasst.

22. Kosmetische Zusammensetzung wie in einem der Ansprüche 1 bis 21 beansprucht, **dadurch gekennzeichnet, dass** die wässrige Phase ein Gemisch von Wasser und wassermischbarem organischem Lösungsmittel umfasst.

23. Kosmetische Zusammensetzung wie in einem der Ansprüche 1 bis 22 beansprucht, **dadurch gekennzeichnet, dass** sie 1 bis 95 Gew.-% wässrige Phase, bezogen auf das Gesamtgewicht der Zusammensetzung, umfasst.

24. Kosmetische Zusammensetzung wie in einem der Ansprüche 1 bis 23 beansprucht, **dadurch gekennzeichnet, dass** sie zusätzlich mindestens eine Fettphase umfasst.

25. Kosmetische Zusammensetzung wie in Anspruch 24 beansprucht, **dadurch gekennzeichnet, dass** die Fettphase mindestens eine Fettsubstanz, welche bei Umgebungstemperatur und bei Atmosphärendruck flüssig ist, und/oder mindestens eine Fettsubstanz, welche bei Umgebungstemperatur und bei Atmosphärendruck fest ist, umfasst.

26. Kosmetische Zusammensetzung wie in Anspruch 25 beansprucht, **dadurch gekennzeichnet, dass** die Fettsubstanz, welche bei Umgebungstemperatur und bei Atmosphärendruck flüssig ist, mindestens ein flüchtiges oder nicht-flüchtiges Öl oder eines von ihren Gemischen umfasst.

27. Kosmetische Zusammensetzung wie in Anspruch 26 beansprucht, **dadurch gekennzeichnet, dass** mindestens ein nicht-flüchtiges Öl 0,01 bis 90 Gew.-%, insbesondere 0,1 bis 85 Gew.-%, bezogen auf das Gesamtgewicht der Fettphase, darstellt.

28. Kosmetische Zusammensetzung wie in einem der Ansprüche 25 bis 27 beansprucht, **dadurch gekennzeichnet, dass** die Fettsubstanz, welche bei Umgebungstemperatur und bei Atmosphärendruck fest ist, aus Wachsen, pastenartigen Fettsubstanzen, Gumme und ihren Gemischen ausgewählt ist.

29. Kosmetische Zusammensetzung wie in einem der Ansprüche 25 bis 28 beansprucht, **dadurch gekennzeichnet, dass** die Fettphase mindestens eine feste Fettsubstanz in einem Anteil von 0,01 bis 50 Gew.-%, insbesondere 0,1 bis 40 Gew.-% und insbesondere 0,2 bis 30 Gew.-%, bezogen auf das Gesamtgewicht der Zusammensetzung, umfasst.

30. Kosmetische Zusammensetzung wie in einem der Ansprüche 1 bis 29 beansprucht, **dadurch gekennzeichnet, dass** die Zusammensetzung zusätzlich eine Partikel-Phase in einem Anteil von 0,01 bis 40 Gew.-%, insbesondere 0,01 bis 30 Gew.-% und insbesondere 0,05 bis 20 Gew.-%, bezogen auf das Gesamtgewicht der Zusammensetzung, umfasst.

31. Kosmetische Zusammensetzung wie in Anspruch 30 beansprucht, **dadurch gekennzeichnet, dass** die Partikel-Phase mindestens ein Pigment und/oder mindestens ein perlmuttartiges Mittel und/oder mindestens einen zusätzlichen Füllstoff umfasst.

32. Kosmetische Zusammensetzung wie in einem der Ansprüche 1 bis 31 beansprucht, **dadurch gekennzeichnet, dass** sie in der Form einer direkten oder inversen Emulsion bereitgestellt ist.

33. Kosmetische Zusammensetzung wie in einem der Ansprüche 1 bis 32 beansprucht, **dadurch gekennzeichnet, dass** sie in der Form von Produkten, welche als ein Stift oder in eine Schale gegossen sind, ist.

34. Kosmetische Zusammensetzung wie in einem der Ansprüche 1 bis 33 beansprucht, **dadurch gekennzeichnet, dass** sie in der Form eines Make-ups und/oder Pflegeprodukts für die Haut und/oder die Lippen bereitgestellt ist.

35. Kosmetische Zusammensetzung wie in einem der Ansprüche 1 bis 31 beansprucht, **dadurch gekennzeichnet, dass** sie in der Form eines Produkts zur Pflege und/oder zum Schminken der Nägel bereitgestellt ist.

36. Kosmetische Zusammensetzung wie in einem der Ansprüche 1 bis 31 beansprucht, **dadurch gekennzeichnet, dass** sie in der Form eines Produkts zur Pflege und/oder zum Stylen des Haares bereitgestellt ist.

37. Verwendung eines Stab-Knäuel-Copolymers wie in einem der Ansprüche 1 bis 19 definiert als rheologisches Mittel in einer kosmetischen Zusammensetzung, welche mindestens einen Konservierungsstoff und mindestens eine wässrige Phase umfasst.

**38.** Verfahren zum Schminken und/oder zur Pflege für die Haut, oberflächliche Körpergeschwülste und/oder Haare, umfassend mindestens einen Schritt des Aufbringens einer kosmetischen Zusammensetzung wie in einem der Ansprüche 1 bis 36 definiert.

## Revendications

**1.** Composition cosmétique comprenant dans un milieu physiologiquement acceptable :

- au moins une phase aqueuse,
- au moins un conservateur, et
- au moins un copolymère bloc de type ROD-COIL comprenant au moins une structure bloc polymérique à conformation variable dite COIL liée de manière covalente à au moins une structure bloc polymérique à conformation limitée dite ROD, lesdits copolymères blocs de type ROD-COIL étant mis en oeuvre à des concentrations capables de conférer au milieu physiologiquement acceptable le contenant une viscosité adaptée à la formulation souhaitée.

**2.** Composition cosmétique selon la revendication 1, **caractérisée en ce que** ladite structure polymérique à conformation limitée, dite ROD, incorpore dans la structure chimique au moins un et en particulier au moins deux noyaux aromatiques.

**3.** Composition cosmétique selon la revendication 1 ou 2, **caractérisée en ce que** la conformation limitée de la structure polymérique ROD est assurée en tout ou partie par des interactions $\pi$-$\pi$.

**4.** Composition cosmétique selon l'une quelconque des revendications 1 à 3, **caractérisée en ce que** la distance moyenne entre les extrémités de chaîne dans le bloc COIL, à savoir $<R_0^2>_{coil}$ satisfait à la convention

$$<R_0^2>_{coil} = N L^2$$

où

L représente la longueur d'un monomère, et
N représente le nombre de monomères constituant le bloc.

**5.** Composition cosmétique selon l'une quelconque des revendications 1 à 4, **caractérisée en ce que** le bloc COIL est composé d'un ou plusieurs copolymères ou homopolymères dérivant de la polymérisation radicalaire de monomères comprenant des motifs éthyléniques, vinyliques, allyliques (méth)acrylates et/ou (méth)acrylamides et dérivés.

**6.** Composition cosmétique selon l'une quelconque des revendications 1 à 5, **caractérisée en ce que** le polymère COIL est choisi parmi les :

- copolymères vinyliques/(méth)acrylates, vinyliques/(méth)acrylamides, vinyliques/(méth)acrylates/méthacrylamides, oléfiniques/vinyliques, (méth)acrylates/(méth)acrylamides,
- homopolymères ou copolymères à base d'acétate de vinyle, de styrène, de vinylpyrrolidone, de vinylcaprolactame, de (méth)acrylate de polyoxyde d'éthylène, de (méth)acrylate de stéaryle, de (méth)acrylate de lauryle, de laurate de vinyle, de (méth)acrylate de butyle, de (méth)acrylate d'éthylehexyle, d'acide crotonique, d'acide (méth)acrylique, d'anhydride maléique, d'acide styrène sulfonique, de diméthyldiallylamine, de vinylpyridine, de (méth)acrylate de diméthylaminoéthyle, et de diméthylaminopropyl(méth)acrylamide,
- polycondensats de type polyuréthanne et/ou polyurées, polyesters aliphatiques, polyamides aliphatiques ou de leurs copolymères, comme par exemple polyuréthanne/urée et polyester/amide,
- polymères obtenus par ouverture de cycle, comme les polyéthers de type polyoxyde d'éthylène, polyoxyde de propylène, les polylactides et leurs copolymères polyoxyde d'éthylène/polyoxyde de propylène ; les polyesters comme la polycaprolactone ; les polyoxazolines tels que la poly(2-méthyloxazoline), ou la poly(2-éthyloxazoline),
- (co)polymères de siloxane, tels que les polydiméthylsiloxane (PDMS), et polyméthylphénylsiloxane,

- polymères obtenus par métathèse comme le poly(narbornène) et ses copolymères,
- polymères obtenus par polymérisation cationique tels que les polyvinylalkyléthers,
- les copolymères de ces différents types de polymère comme par exemple les polysiloxane/ polyoxyde d'éthylène, et
- leurs sels et dérivés.

7. Composition cosmétique selon l'une quelconque des revendications 1 à 6, **caractérisée en ce que** la masse en nombre des blocs COIL varie de 500 g/mol à 1 000 000 g/mol, en particulier de 500 g/mol à 800 000 g/mol, et plus particulièrement de 500 g/mol à 500 000 g/mol.

8. Composition cosmétique selon l'une quelconque des revendications 1 à 7, **caractérisée en ce que** la structure polymérique ROD présente une distance moyenne entre les extrémités de chaîne, $<R_0^2>_{rod}$ satisfaisant à la convention :

$$<R_0^2>_{rod} = CN\,L^2$$

où

L représente la longueur d'un monomère,
C représente les restrictions imposées à la chaîne, avec C supérieur à 1, et notamment varie de 4 à 10, et
N représente le nombre de monomères constituant le bloc.

9. Composition selon l'une quelconque des revendications 1 à 8, **caractérisée en ce que** le bloc ROD comprend au moins une structure aromatique de formule :

$$-[Ar]-_n$$

avec :

- n pouvant varier de 2 à 1000, notamment de 5 à 700, et en particulier de 20 à 700, et
- le(s) groupement(s) Ar pouvant représenter un ou plusieurs motif(s) aromatique(s) ou dérivé(s) de motif aromatique,

l'ensemble des motifs aromatiques constituant ladite formule étant de nature chimique identique ou différente.

10. Composition selon l'une quelconque des revendications 1 à 9, **caractérisée en ce que** le bloc ROD est choisi parmi :

- les polyarylènes, les poly(arylène vinylènes), les poly(arylène éthynylènes), les poly(arylèneéthers) et parmi ceux-ci, les poly(paraphénylènes), poly(paraphénylène vinylènes), poly(paraphénylène éthynylènes), poly(paraphénylène éthers), poly(paraphénylène sulfures) et poly(biphénylène éthers),
- les polyquinolines, polyquinoxalines, les polypyridines, les poly(pyridine vinylènes), les poly(naphthylène vinylènes), les polythiophènes, poly(thiophène vinylènes), poly(thiophène éthynylènes), poly(bis thiophènes), les polyfuranes, polypyrroles, polyanilines, polybenzimidazoles, polybenzothiazoles, polybenzooxazoles, polybenzobisazoles, polyamides aromatiques, polyhydrazides aromatiques, polyazométhines aromatiques, polyesters aromatiques, polyimides aromatiques, polycarbazoles, polyfluorènes, polyanthracènes, et
- leurs copolymères.

11. Composition selon l'une quelconque des revendications 1 à 10, **caractérisée en ce que** le bloc ROD est choisi parmi :

- poly[2,5 bis[4 méthoxyphényl)oxycarbonyl]styrène,
- poly (4'-oxy-biphényle),
- poly(phénylène benzobisthiazole),
- poly(1,4-(2,5 dihexyl phénylène),
- poly(1,4-(2,5 dihexyl)phénylène)éthynylène),
- poly(1,4-(2,5 dihexyl)phénylène)vinylène),
- poly(phénylène benzobisthiazole),

- poly(3-dodécylthiophène),
- poly(3-octylthiophène),
- poly [1,4-(2,5 (polydiméthylsiloxane) phénylène)], et
- leurs copolymères.

**12.** Composition selon l'une quelconque des revendications 1 à 11, **caractérisée en ce que** le bloc ROD est hydrosoluble ou hydrodispersible.

**13.** Composition selon l'une quelconques des revendications 1 à 12, **caractérisée en ce que** le bloc ROD possède un ou plusieurs groupements solubilisants capables de lui procurer la dispersibilité ou solubilité dans une phase aqueuse.

**14.** Composition selon la revendication 13, **caractérisée en ce que** le groupement solubilisant est choisi parmi les motifs $-COOH$, $-CH_2COOH$, $-(CH_2)_2COOH$, $-(CH_2)_3COOH$, $-OH$, $-CH_2OH$, $-(CH_2)_6OH$, $-(CH_2)SO_3H$, $-(CH_2)_2SO_3H$, $-(CH_2)_3SO_3H$, $-O(CH_2)_3SO_3H$, $-(CH_2)_2N(CH_3)(CH_3)$, $-N(CH_3)(CH_3)$, $-O(CH_2)_3N(CH_2CH_3)_2$, $-(CH_2)_2N(CH_2CH_3)_2$, $-[(CH_2)_2O]_xCH_2CH_2OH$, $-[(CH_2)_2O]_xCH_2CH_2OCH_3$, ou $-[(CH_2)_2O]_xCH_2CH_2OCH_2CH_3$ avec x figurant un nombre moyen compris entre 0 et 200, leurs sels de sodium et d'amine, et en particulier de triéthylamine ou de tributylamine.

**15.** Composition cosmétique selon l'une quelconque des revendications 1 à 14, **caractérisée en ce que** la masse en nombre des blocs ROD varie de 200 g/mol à 1 000 000 g/mol, en particulier de 250 g/mol à 800 000 g/mol, et plus particulièrement de 250 g/mol à 500 000 g/mol.

**16.** Composition cosmétique selon l'une quelconque des revendications 1 à 15, **caractérisée par le fait que** les blocs ROD sont présents à raison d'au moins 10 %, en particulier d'au moins 15 %, notamment d'au moins 30 %, et en particulier à raison d'au plus 90 %, notamment d'au plus 85 %, voire d'au plus 80 % en poids par rapport au poids total du copolymère.

**17.** Composition cosmétique selon l'une quelconque des revendications 1 à 16, **caractérisée en ce que** la masse globale en nombre du copolymère ROD-COIL varie de 700 g/mol à 1 000 000 g/mol, en particulier de 1 000 g/mol à 800 000 g/mol, et plus particulièrement de 2 000 g/mol à 500 000 g/mol.

**18.** Composition cosmétique selon l'une quelconque des revendications 1 à 17, **caractérisée en qu'**elle comprend au moins un copolymère blocROD-COIL choisi parmi :

• les diblocs : ROD-b-COIL

- polydiméthylsiloxane -b- poly[2,5 bis[4 méthoxyphényl)oxycarbonyl] styrène
- poly (éthylène/butylène)-b-poly (4'-oxy-biphényle)
- polyester alkyde à chaînes carbonées C12-b-poly(biphényl-4'-biphényl carboxylate)
- poly laurate de vinyle-b-poly(phénylène benzobisthiazole)
- poly(isoprène)-b)- poly(1,4-(2,5 dihexyl phénylène), et

• les triblocs : COIL-b-ROD-b-COIL

- polydiméthylsiloxane -b-poly(1,4-(2,5 dihexyl phénylène) éthynylène)-b-polydiméthylsiloxane
- poly(éthylène/butylène)-b-poly(1,4-(2,5 dihexyl phénylène) éthynylène -b-poly (éthylène/butylène)
- polyester alkyde à chaînes carbonées C12-b- poly(biphényl-4'-biphényl carboxylates) -b- polyester alkyde à chaînes carbonées C12
- poly laurate de vinyle-b-poly (4'-oxy-biphényl) -b-poly laurate de vinyle
- poly(oléyl acrylate)-b-poly(2, 5 (hexyl thiophène)-b- poly(oléyl acrylate),et
- leurs sels ou dérivés.

**19.** Composition cosmétique selon l'une quelconque des revendications 1 à 18, **caractérisée en ce qu'**elle comprend de 0,5 à 90 % en poids, en particulier de 0,7 à 85 % en poids, et plus particulièrement de 0,8 à 75 % en poids de copolymère(s) blocs ROD-COIL par rapport au poids total de la composition.

**20.** Composition cosmétique selon l'une quelconque des revendications 1 à 19, **caractérisée en ce qu'**elle comprend au moins un conservateur choisi parmi les esters de l'acide parahydroxybenzoïque, le phénoxyéthanol, les libérateurs

de formol, le benzoate de sodium, le digluconate de chlorhexidine, les bromures d'alkyltriméthylammonium et leurs mélanges et les sels d'ammonium quaternaires de polymère, tels que le polyquaternium-10, et leurs mélanges.

**21.** Composition cosmétique selon l'une quelconque des revendications 1 à 20, **caractérisée en ce qu'**elle comprend de 0,001 à 10 % en poids de conservateur par rapport au poids total de la composition.

**22.** Composition cosmétique selon l'une quelconque des revendications 1 à 21, **caractérisée en ce que** la phase aqueuse comprend un mélange d'eau et de solvant organique miscible à l'eau.

**23.** Composition cosmétique selon l'une quelconque des revendications 1 à 22, **caractérisée en ce qu'**elle comprend de 1 à 95 % en poids de phase aqueuse, par rapport au poids total de la composition.

**24.** Composition cosmétique selon l'une quelconque des revendications 1 à 23, **caractérisée en ce qu'**elle comprend en outre au moins une phase grasse.

**25.** Composition cosmétique selon la revendication 24, **caractérisée en ce que** ladite phase grasse contient au moins un corps gras liquide à température ambiante et à pression atmosphérique et/ou au moins un corps gras solide à température ambiante et à pression atmosphérique.

**26.** Composition cosmétique selon la revendication 25, **caractérisée en ce que** ledit corps gras liquide à température ambiante et à pression atmosphérique comprend au moins une huile volatile ou non volatile ou un de leurs mélanges.

**27.** Composition cosmétique selon la revendication 25 ou 26, **caractérisée en ce que** ledit corps gras liquide à température ambiante et à pression atmosphérique représente de 0,01 à 90 % en poids, notamment de 0,1 à 85 % en poids par rapport au poids total de la phase grasse.

**28.** Composition cosmétique selon l'une quelconque des revendications 25 à 27, **caractérisée en ce que** ledit corps gras solide à température ambiante et à pression atmosphérique est choisi parmi les cires, les corps gras pâteux, les gommes et leurs mélanges.

**29.** Composition cosmétique selon l'une quelconque des revendications 25 à 28, **caractérisée en ce que** ladite phase grasse contient au moins un corps gras solide à raison de 0,01 à 50 % en poids, notamment de 0,1 à 40 % en poids, et en particulier de 0,2 à 30 %, en poids par rapport au poids total de la composition.

**30.** Composition cosmétique selon l'une quelconque des revendications 1 à 29, **caractérisée en ce que** ladite composition comprend en outre une phase particulaire à raison de 0,01 à 40 % en poids, notamment de 0,01 à 30 % en poids, et en particulier de 0,05 à 20 % en poids, par rapport au poids total de ladite composition.

**31.** Composition cosmétique selon la revendication 30, **caractérisée en ce que** ladite phase particulaire comprend au moins un pigment et/ou au moins une nacre et/ou au moins une charge complémentaire.

**32.** Composition selon l'une quelconque des revendications 1 à 31, **caractérisée en ce qu'**elle se présente sous forme d'une émulsion directe ou inverse.

**33.** Composition selon l'une quelconque des revendications 1 à 32, **caractérisée en ce qu'**elle se présente sous la forme de produits coulés en stick ou en coupelle.

**34.** Composition selon l'une quelconque des revendications 1 à 33, **caractérisée en ce qu'**elle se présente sous la forme d'un produit de maquillage et/ou de soin pour la peau et/ou les lèvres.

**35.** Composition selon l'une quelconque des revendications 1 à 31, **caractérisée en ce qu'**elle se présente sous la forme d'un produit de soin et/ou de maquillage des ongles.

**36.** Composition selon l'une quelconque des revendications 1 à 31, **caractérisée en ce qu'**elle se présente sous la forme d'un produit de soin et/ou de coiffage du cheveu.

**37.** Utilisation d'un copolymère ROD-COIL tel que défini dans l'une quelconque des revendications 1 à 19 à titre d'agent rhéologique, dans une composition cosmétique comprenant au moins un conservateur et au moins une phase

aqueuse.

**38.** Procédé de maquillage et/ou de soin de la peau, des phanères et/ou des cheveux comprenant au moins une étape d'application d'une composition cosmétique telle que définie selon l'une quelconque des revendications 1 à 36.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9947570 A
- EP 542669 A
- EP 787730 A
- EP 787731 A
- WO 9608537 A


### Non-patent literature cited in the description


- **U. Scherf.** *Topics in Current Chemistry,* 1999, vol. 204, 163-222
- chime et physicochimie des polymères. Dunod, 2002
- *Macromol. Rapid Commun.,* 1998, vol. 19, 275-281
- *Advanced Materials,* 2001, vol. 13 (18), 1363
- **M.B. Smith ; J. March.** Advanced Organic Chemistry. Wiley Interscience
- *Macromolecules,* 1999, vol. 32, 7688-7691
- Encyclopedia of Chemical Technology. Wiley, 1979, vol. 22, 333-432
- ENCYCLOPEDIA OF CHEMICAL TECHNOLOGIE. 347-377